(19) Europäisches Patentamt
European Patent Office
Office européen des brevets

(11) Publication number: 0 341 603 A2

# (12) EUROPEAN PATENT APPLICATION

(21) Application number: 89108140.8

(51) Int. Cl.4: C07K 7/08 , A61K 37/02

(22) Date of filing: 05.05.89

(30) Priority: 09.05.88 US 191644

(43) Date of publication of application: 15.11.89 Bulletin 89/46

(84) Designated Contracting States: ES GR

(71) Applicant: ABBOTT LABORATORIES
One Abbott Park Road
Abbott Park, IL 60064-3500(US)

(72) Inventor: Rockway, Todd W.
W38 Oakdale Lane
Mundelein, Illinois 60060(US)
Inventor: Kiso, Yoshiaki
15-26-Inaba-cho, Ibaraki-shi
Osaka 567(JP)
Inventor: Davidsen, Steven K.
840 Banbury Road
Mundelein, Illinois 60060(US)
Inventor: Lucas, Scott D.
302 Hojem
Grayslake, Illinois 60030(US)
Inventor: Thomas, A. Mitchell
38660 Shagbark Lane
Wadsworth, Illinois 60083(US)
Inventor: Von Geldern, Thomas W.
4209 W. Solon Road
Richmond, Illinois 60071(US)

(74) Representative: Modiano, Guido et al
MODIANO, JOSIF, PISANTY & STAUB
Modiano & Associati Via Meravigli, 16
I-20123 Milan(IT)

## (54) Atrial peptide derivatives.

(57) Atrial peptides comprising the amino acid sequence as follows:

S – S

$$R_1-R_2-R_3-R_4-R_5-R_6-R_7-R_8-R_9-R_{10}-R_{11}-R_{12}-R_{13}$$

wherein $R_1$ is hydrogen, Ser, Cit, SerSer, Arg, Carbamoyl Ser, Guanido-Ser-Ser, Amcca, Guanido-Amcca, Lys, ArgArg, Ser-Arg, Ser-Lys, Acetyl-Ser-Ser, D-Ser-Ser, Ser-D-Ser, Guanido-Aocta, Aunda, Aocta, Ahexa, HSer, Apenta, Papaa, Abuta, Mamba, Orn, D-Orn or Acetyl-apenta, Acetyl-Ahexa, Guanido;
$R_2$ is Cys, D-Cys or Pen;
$R_3$ is L-Phe, D-Ala, L-Ala, D-Val, D-Cha, Sar, D-Phg, Aib, β-Ala or Gly;
$R_4$ is Phe, Trp, D-Tic, L-Tic, N-MePhe, D-Ala, Tyr, 2-Thi, DimeDOPA or DOPA;
$R_5$ is Gly-Gly, Ala-Gly, D-Ala-Gly, Gly-Ala, Amcca, Mapaa or Papaa;
$R_6$ is Arg, D-Arg or Cit;
$R_7$ is Ile, Leu, D-Leu or Met;
$R_8$ is Asp, Asn, β-methylAsp, cycloAsp, Gly or Ser;
$R_9$ is Arg, D-Arg or Ala;
$R_{10}$ is Ile, Leu or Phe;
$R_{11}$ is Cys, D-Cys, HCys, Cys-4-thiomethylproline, or trans-4-thiomethylproline;
$R_{12}$ is absent, Phe, D-Ala-Phe, D-Phe, Cha, Pro, D-Pro, 2-Thi, Aic, D-Tic, trans-4-FPro, L-Tic, Leu, Ile or β-NAl;
$R_{13}$ is OH, Arg, Arg-$NH_2$, D-Arg, Gln, Lys, HomoArg, or Gly; or pharmaceutically acceptable salts, esters or

EP 0 341 603 A2

amides thereof.

# ATRIAL PEPTIDE DERIVATIVES

## Technical Field:

The present invention relates to derivatives of atrial natriuretic polypeptides which exhibit useful hypotensive, natriuretic, diuretic, renovasodilating, renoprotective and vasorelaxant activity.

## Background Art:

Recently, alpha-human atrial natriuretic polypeptide (alpha-hANP) has been isolated from human atrium and identified as a polypeptide consisting of 28 amino acids having the following formula:

Ser Leu Arg Arg Ser Ser Cys Phe Gly Gly Arg Met Asp-

S———S

Arg Ile Gly Ala Gln Ser Gly Leu Gly Cys Asn Ser Phe Arg Tyr

It has been reported that the diuretic action of the alpha-hANP was about 1000 times as strong as that of furosemide which is used as a hypotensive diuretic. Others have found that alpha-hANP increases urinary sodium and urinary output.

However, alpha-hANP or derivatives thereof have not been commercially available and research for ANP derivatives which are chemically and enzymatically stable, have long duration and stronger action than alpha-hANP has been actively pursued.

Heretofore, hypertension has been classified into hypertension having clear factor disease and one having obscure factor disease. The former is classified as secondary hypertension and involves mainly those caused by kidney disease, endocrine disease, toxemia of pregnancy, arterial embolism, central nervous system disease and the like. Alternatively, the later hypertension having obscure factor disease is classified as essential hypertension, in which 80-90% of hypertension patients have been classified. Though the conditions of the secondary hypertension can be improved by treating the factor disease, the treatment of conditions of the essential hypertension having obscure factor disease have been effected by, for example, the administration of a hypotensive diuretic or vasodilator as symptomatic therapy. Recently, it has been suggested that the above alpha-hANP is one of the materials which is related to the cause of heart disease or essential hypertension and research on essential hypertension concerning alpha-hANP has drawn attention.

## Brief Description of the Invention

In accordance with the present invention, novel peptides are provided which exhibit useful hypotensive, natriuretic, diuretic, renovasodilating, renoprotective and vasorelaxant activity. These biologically active peptides have the following amino acid sequence:

S – S

$$R_1-R_2-R_3-R_4-R_5-R_6-R_7-R_8-R_9-R_{10}-R_{11}-R_{12}-R_{13}$$

(Formula I)

wherein $R_1$ is hydrogen, Ser, Cit, SerSer, Arg, Carbamoyl Ser, Guanido-Ser-Ser, Amcca, Guanido-Amcca, Lys, ArgArg, Ser-Arg, Ser-Lys, Acetyl-Ser-Ser, D-Ser-Ser, Ser-D-Ser, Guanido-Aocta, Aunda, Aocta, Ahexa, HSer, Apenta, Papaa, Abuta, Mamba, Orn, D-Orn or Acetyl-apenta, Acetyl-Ahexa, Guanido;
$R_2$ is Cys, D-Cys or Pen;
$R_3$ is L-Phe, D-Ala, L-Ala, D-Val, D-Cha, Sar, D-Phg, Aib, $\beta$-Ala or Gly;
$R_4$ is Phe, Trp, D-Tic, L-Tic, N-MePhe, D-Ala, Tyr, 2-Thi, DimeDOPA or DOPA;
$R_5$ is Gly-Gly, Ala-Gly, D-Ala-Gly, Gly-Ala, Amcaa, Mapaa or Papaa;
$R_6$ is Arg, D-Arg or Cit;
$R_7$ is Ile, Leu, D-Leu or Met;
$R_8$ is Asp, Asn, $\beta$-methylAsp, cycloAsp, Gly or Ser;
$R_9$ is Arg, D-Arg or Ala;
$R_{10}$ is Ile, Leu or Phe;
$R_{11}$ is Cys, D-Cys, HCys, Cys-4-thiomethylproline, or trans-4-thiomethylproline;
$R_{12}$ is absent, Phe, D-Ala-Phe, D-Phe, Cha, Pro, D-Pro, 2-Thi, Aic, D-Tic, trans-4-FPro, L-Tic, Leu, Ile or $\beta$-NAl;
$R_{13}$ is OH, Arg, Arg-$NH_2$, D-Arg, Gln, Lys, HomoArg, or Gly; or pharmaceutically acceptable salts, esters or amides thereof.

In the above peptide structure, the amino acid components are designated by conventional abbreviations as follows:

| Amino Acid | Abbreviated Designation |
|---|---|
| 4-Aminobutanoic Acid | Abuta |
| 6-Aminohexanoic Acid | Ahexa |
| 2-Aminoisobutyric Acid | Aib |
| 2-Aminoindane-2-carboxylic Acid | Aic |
| Alanine | Ala |
| trans-4-Aminomethylcyclohexyl-1-carboxylic Acid | Amcca |
| 8-Aminooctanoic Acid | Aocta |
| 5-Aminopentanoic Acid | Apenta |
| 3-Aminopropanoic Acid | β-Ala |
| Arginine | Arg |
| Asparagine | Asn |
| Aspartic Acid | Asp |
| Asparagine and/or Aspartic Acid | Asx |
| 11-Aminoundecanoic Acid | Aunda |
| Cyclohexylalanine | Cha |
| Citrulline | Cit |
| Aspartic Acid gamma-lactam | cycloAsp |
| Cysteine | Cys |
| 3,4-Dimethoxyphenylalanine | DimeDOPA |
| 3,4-Dihydroxyphenylalanine | DOPA |
| Glutamine | Gln |
| Glycine | Gly |
| 2-amino-w-guanido-hexanoic acid | HArg |
| 3-((S)-Hydroxymethyl)-3-aminopropanoic Acid | HSer |
| 3-amino-4-mercaptobutanoic acid | HCys |
| Isoleucine | Ile. |
| Leucine | Leu |
| Lysine | Lys |
| 3-Aminophenylacetic Acid | Mapaa |
| meta-amino-methylbenzoic acid | Mamba |
| Methionine | Met |
| Aspartic Acid, β-methyl ester | β-methylAsp |
| N-Methylphenylalanine | N-MePhe |
| β-Naphthylalanine | β-Nal |
| 4-Aminophenylacetic Acid | Papaa |
| Penicillamine | Pen |
| Phenylalanine | Phe |
| Phenylglycine | Phg |
| Proline | Pro |
| Sarcosine | Sar |
| Serine | Ser |
| 2-Thienylalanine | 2-Thi |
| Tetrahydroisoquinoline-3-carboxylic Acid | Tic |
| trans-4-Phenylproline | trans-4-FPro |
| Tryptophan | Trp |
| Tyrosine | Tyr |

Unless expressly stated otherwise, the optically active amino acids always have the L-configuration.

The term "aryl" as used herein means phenyl, benzyl or naphthyl groups.

Examples of pharmaceutically acceptable, non-toxic acid addition salts are salts formed with inorganic acids such as hydrochloric acid, hydrobromic acid, phosphoric acid, sulfuric acid and perchloric acid or with organic acids such as acetic acid, oxalic acid, maleic acid, malic acid, tartaric acid, citric acid, succinic acid or malonic acid. Other pharmaceutically acceptable salts include inorganic nitrate, sulfate, acetate, malate, formate, lactate, tartrate, succinate, citrate, p-toluenesulfonate, and the like or metal salts such as sodium, potassium or calcium salts or ammonium or amino salts such as ammonium, triethylamine salts, and the like and they may be prepared according to conventional methods.

Examples of pharmaceutically acceptable, non-toxic esters of the compound of formula I include $C_1$ to $C_6$ alkyl esters wherein the alkyl group is straight or branched chain. Acceptable esters also include $C_5$ to $C_7$ cycloalkyl esters. $C_1$ to $C_4$ alkyl esters are preferred. Esters of the compound of formula I may be prepared according to conventional methods.

Examples of pharmaceutically acceptable, non-toxic amides of the compound of formula I include amides derived from ammonia, primary $C_1$ to $C_6$ alkyl amines and secondary $C_1$ to $C_6$ dialkyl amines wherein the alkyl groups are straight or branched chain. In the case of secondary amines the amine may also be in the form of a 5 or 6 membered heterocycle containing one nitrogen atom. Amides derived from ammonia, $C_1$ to $C_3$ alkyl primary amides and $C_1$ to $C_2$ dialkyl secondary amides are preferred. Amides of the compound of formula I may be prepared according to conventional methods.

The daily dosage of the compounds of the present invention may be suitably defined according to the conditions of the patient by those skilled in the art but generally may be administered in an amount of about 0.2-250 mg/kg body weight. Dosage unit compositions may contain such amounts of submultiples thereof to make up the daily dose.

The amount of active ingredient that may be combined with carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents, such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The novel polypeptides and salts thereof of the invention can be utilized effectively as vasorelaxant agents, diuretics, natriuretic agents, renovasodilators or renoprotective agents. Accordingly, the present invention further relates to vasorelaxant, diuretic, natriuretic, renovasodilating or renoprotective compositions comprising a novel polypeptide having the general formula I or salts thereof as an active component.

The polypeptides of the invention may be prepared by a synthetic method of elongation of a peptide chain through condensation of one amino acid by one, or by a method of coupling fragments consisting of two or several amino acids, or by a combination of these methods in accordance with conventional peptide synthesis methods.

The condensation of two amino acids, the condensation of an amino acid with a peptide or the condensation of one peptide with another peptide may be effected in accordance with conventional condensation methods such as azide method, mixed acid anhydride method, DCC (dicyclohexylcarbodiimide) method, active ester method (p-nitrophenyl ester method, N-hydroxysuccinic acid imide ester method, cyanomethyl ester method and the like), Woodward reagent K method, DCC-HOBT(1-hydroxy-benzotriazole) method and the like. These condensation reactions may be done by either solution methods or solid phase synthetic methods. When the peptide chain is elongated by solid phase method, the C-terminal amino acid was linked to an insoluble carrier. As the insoluble carrier, any of which can produce a detachable bond by reacting with a carboxyl group in a C-terminal amino acid may be used, and the examples thereof involve, for example, halomethyl resins such as chloromethyl resin, bromomethyl resin and the like, hydroxy-methyl resin, aminomethyl resin, benzhydrylamine resin, and t-alkyloxycarbonyl hydrazide resin.

As conventional polypeptide synthesis, branched chain amino and carboxyl groups at alpha and omega positions in amino acids may be protected/deprotected if necessary. The protecting groups for amino groups which can be used involve, for example, benzyloxycarbonyl (Z), o-chlorobenzyloxycarbonyl ((2-Cl)-Z), p-nitrobenzyloxycarbonyl ($Z(NO_2)$), p-methoxybenzyloxycarbonyl (Z(OMe)), t-butoxycarbonyl (Boc), t-amyloxycarbonyl (Aoc), isobornyloxycarbonyl, admantyloxycarbonyl, 2-(4-biphenyl)-2- propyloxycarbonyl (Bpoc), 9-fluorenyl-methoxycarbonyl (Fmoc), methylsulfonylethoxycarbonyl (Msc), trifluoroacetyl, phthalyl, formyl, 2-nitrophenylsulfenyl (Nps), diphenylphosphinothioyl (Ppt), and dimethylphosphinothioyl (Mpt).

The examples of protecting groups for carboxyl groups involve, for example, benzyl ester (OBzl), cyclohexyl ester, 4-nitrobenzyl ester ($OBzlNO_2$), t-butyl ester (OtBu), 4-pyridylmethyl ester (OPic) and the like.

In the course of the synthesis of the present peptides, specific amino acids having functional groups other than amino and carboxyl groups in the branched chain such as arginine, cysteine, serine, and the like may be protected, if necessary, with suitable protecting group. It is preferable that for example, the guanidino group ($N^G$) in arginine may be protected with nitro,p-toluenesulfonyl (Tos), benzyloxycarbonyl (Z), adamantyloxycarbonyl (Adoc), p-methoxybenzenesulfonyl, 4-methoxy-2,6-dimethylbenzenesulfonyl (Mds), 1,3,5-trimethylphenylsulfonyl (Mts) and the like, and the thiol group in cysteine may be protected with benzyl, p-methoxybenzyl, triphenylmethyl, acetamidomethyl, ethylcarbamyl, 4-methylbenzyl (4-MeBzl), 2,4,6-trimethylbenzyl (Tmb) and the like, and the hydroxyl group in serine may be protected with benzyl (Bzl), t-butyl, acetyl, tetrahydropyranyl and the like.

The compounds of the invention are prepared by standard solid phase peptide synthesis conditions as described in "Solid Phase Peptide Synthesis" by John M. Stewart and Janis D. Young, Second Edition (1984) and illustrated in Examples 1 and 2 in the experimental section.

The compounds of the invention may also be prepared by partial solid phase synthesis, fragment condensation methods and classical solution methods as exemplified by the methods described in "Peptide Synthesis", Second Edition, M. Bodanszky, Y. S. Klausner, and M. A. Ondetti (1976).

The foregoing may be better understood by reference to the following examples which are provided for illustration and not limitation of the practice of the invention.

## Example 1

Arginyl ($N^G$-Tos)-Cysteinyl(4-MeBzl)-D-Alanyl-Phenylalanyl-Glycyl-Glycyl-Arginyl($N^G$-Tos)-Leucyl-Aspartyl($\beta$-Cyclohexyl)-Arginyl($N^G$-Tos)-Isoleucyl-Cysteinyl(4-MeBzl)-Phenylalanyl-Arginyl($N^G$-Tos)-p-Methyl-Benzhydrylamine protected peptide resin.

0.5-1.0 g. p-Methyl-Benzhydrylamine resin was placed in a solid phase peptide synthesis vessel and amino acids were attached to the peptide resin sequentially in the following order: Boc-Arg($N^G$-Tos), Boc-Phe, Boc-Cys(4-MeBzl), Boc-Ile, Boc-Arg($N^G$-Tos), Boc-Asp($\beta$-cyclohexyl), Boc-Leu, Boc-Arg($N^G$-Tos), Boc-Gly, Boc-Gly, Boc-Phe, Boc-D-Ala, Boc-Cys(4-MeBzl), Boc-Arg($N^G$-Tos), according to the protocol outlined in Agenda A to yield the protected peptide resin: Arginyl($N^G$-Tos)-Cysteinyl(4-MeBzl)-D-Alanyl-Phenylalanyl-Glycyl-Glycyl-Arginyl($N^G$-Tos)-Leucyl-Aspartyl($\beta$-cyclohexyl)-Arginyl($N^G$-Tos)-Isoleucyl-Cysteinyl(4-MeBzl)-Phenylalanyl-Arginyl ($N^G$-Tos)-p-Methyl-Benzhydrylamine resin. Following the synthesis, the protected peptide resin was removed from the reaction vessel by washing the resin three times with 20 ml DMF into a 60 ml sintered glass funnel, followed by washing the resin three times with 30 ml $CH_2Cl_2$. The resin was dried in vacuo three hours, then weighed.

## Agenda A

1. Deblock: 50% Trifluoroacetic acid (TFA) in $CH_2Cl_2$ containing 2% Anisole, 0.5% Ethanedithiol (v v v).

2. Neutralization: 10% Diisopropylethylamine (DIEA) in $CH_2Cl_2$ (v/v).

3. Single Coupling: 0.2 M Boc-amino acid derivative in N,N-dimethylformamide (DMF), 0.2 M diisopropylcarbodiimide in $CH_2Cl_2$, reaction time 60 minutes.

4. Resin Clean Up: 10% DIEA in $CH_2Cl_2$.

5. Single Coupling: 0.2 M Boc-amino acid derivative (same as Step 3) in DMF; 0.2 M diisopropylcarbodiimide in $CH_2Cl_2$, reaction time 60 minutes.

6. Resin cleanup: 10% DIEA in $CH_2Cl_2$

7. Cap: 0.3 M Acetylimidazole in DMF.

8. Go to next amino acid residue.

9. Upon attachment of the final amino acid to the growing peptide chain, the protecting group (t-Boc) is removed as in Step 1.

## Example 2

Arginyl-Cysteinyl-D-Alanyl-Phenylalanyl-Glycyl-Glycyl-Arginyl-Leucyl-Aspartyl-Arginyl-Isoleucyl-Cysteinyl-Phenylalanyl-Arginyl-Carboxamide Cyclic Disulfide

0.8 g protected peptide resin of Example 1 was treated with 2 ml anisole and 15 ml liquid hydrogen fluoride (HF) for 60 minutes at 0 °C. The HF and anisole was removed from the resin in vacuo and the resin was washed with 2 x 25 ml portions of ethyl ether or ethyl acetate under a nitrogen atmosphere. The crude peptide was extracted from the resin by treatment with 6 x 50 ml portions of deoxygenated 20% aqueous acetic acid. The acetic acid solution was diluted to 1,500 ml with distilled water and the pH of the solution was adjusted to 7.2 with 80 ml concentrated ammonium hydroxide. The solution was oxidized with an excess of 0.01N $I_2$(EtOH). The resultant yellow solution was stirred for 60 minutes at room temperature while the pH was maintained at 7.2. The excess iodine was removed from the solution by first adjusting the pH to 5.5 with 50 ml glacial acetic acid and then adding 3-6 ml of 0.01N sodium thiosulfate solution. The combined aqueous solution was applied to a column containing 300 g XAD-16 molecular adsorbent resin. The sample was desalted by first washing the column with 3 l distilled water. The sample was eluted from the column with 1,500 ml 50% aqueous ethanol. The combined ethanol fractions were concentrated to approximately 100 ml in vacuo, taken up in an additional 100 ml distilled water and lyophilized to a dry amorphous powder. The dry powder was purified by High Performance Liquid Chromatrography (HPLC) using 0.1% aqueous trifluoroacetic acid and acetonitrile as organic modifier. The preparative HPLC was performed using a Dynamax (RAININ) $C_{18}$ Reverse-phase Column 2.1 cm i.d. x 25 cm length and flow rate of 12-15 ml/min. The sample was purified by gradient elution. Analytical HPLC was performed using a VYDAC 218 TP 10u, $C_{18}$ Reverse-phase Column 0.46 cm i.d. x 20 cm length. There was obtained 28 mg (6%) of the title compound.

Amino acid Analysis: Asx(1.0), Gly(2.0), Cys(1.5), Ile(1.1), Phe(2.0), Arg(4.0); Ala(1.0), Leu(1.1).

FAB $(M+H)^+$ at M/Z: 1667. The 300 MHz $^1$H NMR spectrum is consistent with the proposed structure.

## Example 3

Arginyl-Cysteinyl-Phenylalanyl-D-Alanyl-Glycyl-Glycyl-Arginyl-Isoleucyl-Aspartyl-Arginyl-Isoleucyl-Cysteinyl-Phenylalanyl-Arginyl Cyclic Disulfide

0.40 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-D-Ala, Boc-Phe, Boc-Cys(4-MeBzl), Boc-Arg($N^G$-Tos), was treated with HF (liquid) and purified as described in Example 2. The title compound was obtained as a white powder, 25 mg.
FAB $(M+H)^+$ at M/Z: 1668.

Amino acid analysis: Arg(4.0), Asx(1.1), Cys(1.8), Gly(2.2), Ile(2.1), Ala(1.0), Phe(2.0).

The 300 MHz $^1$H NMR spectrum is consistent with the proposed structure.

## Example 4

Arginyl-Cysteinyl-D-Alanyl-Phenylalanyl-Glycyl-Glycyl-Arginyl-Leucyl-Aspartyl-Arginyl-Isoleucyl-Cysteinyl-Phenylalanyl-Arginyl Cyclic Disulfide

0.40 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Leu, Boc-Arg($N^G$-Tos), Boc-Gly, Boc-Gly, Boc-Phe, Boc-D-Ala, Boc-Cys(4-MeBzl), Boc-Arg($N^G$-Tos), was treated with HF (liquid) and purified as described in Example 2. The title compound was obtained as a white powder, 18 mg.
FAB $(M+H)^+$ at M/Z: 1668.

Amino acid analysis: Arg(4.0), Asx(1.0), Cys(1.7), Gly(2.1), Ile(1.1), Leu(1.0), Phe(2.1), Ala(1.0).

The 300 MHz $^1$H NMR spectrum is consistent with the proposed structure.

## Example 5

Seryl-Seryl-Penicillaminyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Pen(4-MeBzl), Boc-Ser(Bzl), Boc-Ser(Bzl), was treated with HF (liquid) and purified as described in Example 2. The title compound was obtained as a white powder, 25 mg.
FAB $(M+H)^+$ at M/Z: 1714.

Amino acid analysis: Arg(3.0), Asx(1.1), Cys(0.2), Gly(1.9), Ile(1.9), Phe(2.0), Ser(1.5), Ala(0.9).

The 300 MHz $^1$H NMR is consistent with the proposed structure.

Example 6

Seryl-Seryl-Cysteinyl-D-Alanyl-Phenylalanyl-Glycyl-Glycyl-Arginyl-Leucyl-Aspartyl-Arginyl-Isoleucyl-Cysteinyl-Phenylalanyl-Arginyl-Carboxamide Cyclic Disulfide

0.70 g protected peptide resin, prepared from p-methylbenzhydrylamine resin and the amino acids added sequentially in the following order: Boc-Arg($N^G$-Tos), Boc-Phe, Boc-Cys(4-MeBzl), Boc-Ile, Boc-Arg-($N^G$-Tos), Boc-Asp($\beta$-cyclohexyl), Boc-Leu, Boc-Arg($N^G$-Tos), Boc-Gly, Boc-Gly, Boc-Phe, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 23 mg.
FAB $(M+H)^+$ at M/Z: 1685.

Amino acid analysis: Ala(1.1), Arg(3.0), Asx(1.1), Cys(1.6), Gly(2.1), Ile(1.0), Phe(2.1), Ser(1.6), Leu(1.0).

The 300 MHz $^1$H NMR is consistent with the proposed structure.

Example 7

Arginyl-Cysteinyl-Alanyl-Phenylalanyl-Glycyl-Glycyl-Arginyl-Leucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg-($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Leu, Boc-Arg($N^G$-Tos), Boc-Gly, Boc-Gly, Boc-Phe, Boc-Ala, Boc-Cys(4-MeBzl), Boc-Arg($N^G$-Tos), was treated with HF (liquid) and purified as described in Example 2. The title compound was obtained as a white powder, 37 mg.
FAB $(M+H)^+$ at M/Z: 1667.

Amino acid analysis: Arg(4.0), Asx(1.1), Cys(1.8), Gly(2.1), Ile(1.0), Leu(1.1), Phe(2.1), Ala(1.1).

The 300 MHz $^1$H NMR is consistent with the proposed structure.

Example 8

Seryl-Seryl-Cysteinyl-D-Alanyl-Phenylalanyl-Glycyl-Glycyl-Arginyl-Leucyl-Aspartyl-Arginyl-Isoleucyl-Cysteinyl-Cyclohexylalanyl-Arginyl-Carboxamide Cyclic Disulfide

0.75 g protected peptide resin, prepared from benzhydrylamine resin and the amino acids added sequentially in the following order: Boc-Arg($N^G$-Tos), Boc-Cha, Boc-Cys(4-MeBzl), Boc-Ile, Boc-Arg($N^G$-Tos), Boc-Asp($\beta$-cyclohexyl), Boc-Leu, Boc-Arg($N^G$-Tos), Boc-Gly, Boc-Gly, Boc-Phe, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 19 mg.
FAB $(M+H)^+$ at M/Z: 1691

Amino acid analysis: Ala(1.0), Arg(3.0), Asx(1.0), Cys(1.4), Gly(1.8), Ile(0.9), Phe(1.0), Ser(1.5), Leu(1.0).

The 300 MHz $^1$H NMR is consistent with the proposed structure.

## Example 9

Seryl-Seryl-Cysteinyl-Glycyl-Phenylalanyl-Glycyl-Glycyl-Arginyl-Isoleucyl-Aspartyl-Arginyl-Isoleucyl-Cysteinyl-Cyclohexylalanyl-Arginyl-Carboxamide Cyclic Disulfide

0.72 g protected peptide resin, prepared from p-methylbenzhydrylamine resin and the amino acids added sequentially in the following order: Boc-Arg($N^G$-Tos), Boc-Cha, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-Gly, Boc-Cys(4-MeBzl), Boc-Ser(Bzl), Boc-Ser(Bzl), was treated with HF (liquid) and purified as described in Example 2. The title compound was obtained as a white powder, 11 mg.
FAB $(M+H)^+$ at M/Z: 1676.

Amino acid analysis: Arg(3.0), Asx(1.0), Cha(0.7), Cys(1.4), Gly(2.9), Ile(1.8), Phe(1.0), Ser(1.6).

The 300 MHz $^1$H NMR spectrum is consistent with the proposed structure.

## Example 10

Arginyl-Cysteinyl-Glycyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.41 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-Gly, Boc-Cys(4-MeBzl), Boc-Arg($N^G$-Tos), was treated with HF (liquid) and purified as described in Example 2. The title compound was obtained as a white powder, 30 mg.
FAB $(M+H)^+$ at M/Z: 1653.

Amino acid analysis: Arg(3.8), Asx(1.1), Cys(1.7), Gly(3.1), Ile(1.9), Phe(1.9).

The 300 MHz $^1$H NMR spectrum is consistent with the proposed structure.

## Example 11

Seryl-Seryl-Cysteinyl-Glycyl-Phenylalanyl-Glycyl-Glycyl-Arginyl-Isoleucyl-Aspartyl-Arginyl-Isoleucyl-Cysteinyl-Cyclohexylalanyl-Arginyl Cyclic Disulfide

0.60 g protected peptide resin, prepared from N-alpha-t-Boc-Arg($N^G$-Tos) Merrifield resin and the amino acids added sequentially in the following order: Boc-Cha, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-Phe, Boc-Gly, Boc-Cys(4-MeBzl), Boc-Ser(Bzl), Boc-Ser(Bzl), was treated with HF (liquid) and purified as described in Example 2. The title compound was obtained as a white powder, 3 mg.
FAB $(M+H)^+$ at M/Z: 1677.

Amino acid analysis: Arg(2.9), Asx(1.1), Cha(0.8), Cys(1.7), Gly(3.2), Ile(1.9), Phe(1.0), Ser(1.7).

The 300 MHz $^1$H NMR spectrum is consistent with the proposed structure.

## Example 12

Arginyl-Cysteinyl-D-Alanyl-Phenylalanyl-Glycyl-Glycyl-Arginyl-Leucyl-Aspartyl-Arginyl-Isoleucyl-Cysteinyl-Leucyl-Arginyl-Carboxamide Cyclic Disulfide

0.4 g protected peptide resin, prepared from N-alpha-t-Boc-Arg($N^G$-Tos) Merrifield resin and the amino acids added sequentially in the following order: Boc-Leu, Boc-Cys(4-MeBzl), Boc-Ile, Boc-Arg($N^G$-Tos), Boc-Asp(β-cyclohexyl), Boc-Leu, Boc-Arg($N^G$-Tos), Boc-Gly, Boc-Gly, Boc-Phe, Boc-D-Ala, Boc-Cys(4-MeBzl), Boc-Arg($N^G$-Tos), was treated with HF (liquid) and purified as described in Example 2. The title compound was obtained as a white powder, 8 mg.

Amino acid analysis: Arg(4.05), Asx(1.01), Cys(1.5), Gly(2.0), Ile(0.89), Leu(1.92), Phe(1.00), Ala(0.98).

The 300 MHz $^1$H NMR is consistent with the proposed structure.

## Example 13

Seryl-Arginyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.48 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-Phe. Boc-D-Ala, Boc-Cys(4-MeBzl), Boc-Arg($N^G$-Tos), Boc-Ser(Bzl), was treated with HF (liquid) and purified as described in Example 2. The title compound was obtained as a white powder, 43 mg.
FAB $(M+H)^+$ at M/Z: 1755.

Amino acid analysis: Asx(1.1), Ser(0.9), Gly(2.0), Ala(1.1), Cys(1.8), Ile(2.0), Phe(2.1), Arg(4.0).

The 300 MHz $^1$H NMR is consistent with the proposed structure.

## Example 14

D-Seryl-Seryl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.37 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-Ser(Bzl), Boc-(D)Ser(Bzl), was treated with HF (liquid) and purified as described in example 2. The title compound was obtained as a white powder, 31.7 mg.
FAB $(M+H)^+$ at M/Z: 1685.

Amino acid analysis: Asx(1.0), Ser(1.4), Gly(2.1), Ala(1.0), Cys(1.8), Ile(2.0), Phe(1.8), Arg(3.0).

The 300 MHz $^1$H NMR is consistent with the proposed structure.

## Example 15

Seryl-D-Seryl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.47 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-(D)Ser(Bzl), Boc-Ser(Bzl), was treated with HF (liquid) and purified as described in Example 2. The title compound was obtained as a white powder, 38.4 mg.
FAB $(M+H)^+$ at M/Z: 1685.

Amino acid analysis: Asx(0.9), Ser(1.3), Gly(1.9), Ala(0.9), Cys(1.6), Ile(2.0), Phe(1.9), Arg(3.0).

The 300 MHz $^1$H NMR is consistent with the proposed structure.

## Example 16

Lysyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.47 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-Lys(Cl-Cbz), was treated with HF (liquid) and purified as described in example 2. The title compound was obtained as a white powder, 34.1 mg.
FAB $(M+H)^+$ at M/Z: 1639.

Amino acid analysis: Asx(1.0), Gly(2.1), Ala(1.1), Cys(1.7), Ile(1.9), Phe(1.9), Lys(0.9), Arg(3.0).

The 300 MHz $^1$H NMR is consistent with the proposed structure.

## Example 17

Seryl-Seryl-Cysteinyl-Glycyl-Phenylalanyl-Glycyl-Glycyl-Arginyl-Isoleucyl-Aspartyl-Arginyl-Isoleucyl-D-Cysteinyl-Phenylalanyl-Arginyl Cyclic Disulfide

0.58 g protected peptide resin, prepared from N-alpha-t-Boc-Arg($N^G$-Tos) Merrifield resin and the amino acids added sequentially in the following order: Boc-Phe, Boc-D-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-Gly, Boc-Cys(4-MeBzl), Boc-Ser(Bzl), Boc-Ser(Bzl), was treated with HF (liquid) and purified as described in example 2. The title compound was obtained as a white powder, 20.8 mg.
FAB $(M+H)^+$ at M/Z: 1671.

Amino acid analysis: Arg(3.0), Asx(1.0), Cys(1.9), Gly(3.5), Ile(2.0), Phe(2.0), Ser(1.7).

The 300 MHz $^1$H NMR is consistent with the proposed structure.

## Example 18

Seryl-Seryl-Cysteinyl-Glycyl-Phenylalanyl-Glycyl-Glycyl-Arginyl-Isoleucyl-Aspartyl-Arginyl-Isoleucyl-Cysteinyl-D-Phenylalanyl-D-Arginyl Cyclic Disulfide

0.48 g protected peptide resin, prepared from N-alpha-t-Boc-D-Arg($N^G$-Tos) Merrifield resin and the amino acids added sequentially in the following order: Boc-D-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-Gly, Boc-Cys(4-MeBzl), Boc-Ser(Bzl), Boc-Ser(Bzl), was treated with HF (liquid) and purified as described in Example 2. The title compound was obtained as a white powder, 8.0 mg.
FAB $(M+H)^+$ at M/Z: 1672.

Amino acid analysis: Arg(3.0), Asx(0.9), Cys(1.6), Gly(3.3), Ile(1.9), Phe(1.9), Ser(1.4).

The 300 MHz $^1$H NMR is consistent with the proposed structure.

## Example 19

Seryl-Seryl-Cysteinyl-Glycyl-Phenylalanyl-Glycyl-Glycyl-Arginyl-Isoleucyl-Aspartyl-Arginyl-Isoleucyl-Cysteinyl-Phenylalanyl-D-Arginyl Cyclic Disulfide

0.50 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-Gly, Boc-Cys(4-MeBzl), Boc-Ser(Bzl), Boc-Ser(Bzl), was treated with HF (liquid) and purified as described in Example 2. The title compound was obtained as a white powder, 10.0 mg.
FAB $(M+H)^+$ at M/Z: 1671.

Amino Acid Analysis: Arg(3.0), Asx(0.9), Cys(1.7), Gly(3.2), Ile(1.8), Phe(1.8), Ser(1.4).

The 300 MHz $^1H$ NMR is consistent with the proposed structure.

Example 20

Seryl-Seryl-Cysteinyl-Glycyl-Phenylalanyl-Glycyl-Glycyl-Arginyl-Isoleucyl-Aspartyl-Arginyl-Isoleucyl-Cysteinyl-D-Prolyl-Arginyl Cyclic Disulfide

0.55 g protected peptide resin, prepared from N-alpha-t-Boc-Arg($N^G$-Tos) Merrifield resin and the amino acids added sequentially in the following order: Boc-D-Pro, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-Gly, Boc-Cys(4-MeBzl), Boc-Ser(Bzl), Boc-Ser(Bzl), was treated with HF (liquid) and purified as described in Example 2. The title compound was obtained as a white powder, 26.1 mg.
FAB $(M+H)^+$ at M/Z: 1622.

Amino Acid Analysis: Arg(2.6), Asx(0.9), Cys(1.9), Gly(3.2), Ile(2.0), Phe(1.0), Pro(1.4), Ser(1.5).

The 300 MHz $^1H$ NMR is consistent with the proposed structure.

Example 21

Seryl-Seryl-Cysteinyl-Glycyl-Phenylalanyl-Glycyl-Glycyl-Arginyl-Isoleucyl-Aspartyl-Arginyl-Isoleucyl-Cysteinyl-Prolyl-Arginyl Cyclic Disulfide

0.58 g protected peptide resin, prepared from N-alpha-t-Boc-Arg($N^G$-Tos) Merrifield resin and the amino acids added sequentially in the following order: Boc-Pro, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-Gly, Boc-Cys(4-MeBzl), Boc-Ser(Bzl), Boc-Ser(Bzl), was treated with HF (liquid) and purified as described in Example 2. The title compound was obtained as a white powder, 23.8 mg.
FAB $(M+H)^+$ at M/Z: 1622.

Amino Acid Analysis: Arg(3.0), Asx(0.9), Cys(1.7), Gly(3.0), Ile(2.0), Phe(1.0), Pro(1.0), Ser(1.5).

The 300 MHz $^1$H NMR is consistent with the proposed structure.

## Example 22

Seryl-Seryl-Cysteinyl-Glycyl-Phenylalanyl-Glycyl-Glycyl-Arginyl-Isoleucyl-Aspartyl-Arginyl-Isoleucyl-Cysteinyl-D-Phenylalanyl-Arginyl Cyclic Disulfide

0.66 g protected peptide resin, prepared from N-alpha-t-Boc-Arg(N$^G$-Tos) Merrifield resin and the amino acids added sequentially in the following order: Boc-D-Phe, Boc-Cys(4-MeBzl), Boc-Ile, Boc-Arg(N$^G$-Tos), Boc-Asp(β-cyclohexyl), Boc-Ile, Boc-Arg(N$^G$-Tos), Boc-Gly, Boc-Gly, Boc-Phe, Boc-Gly, Boc-Cys(4-MeBzl), Boc-Ser(Bzl), Boc-Ser(Bzl), was treated with HF (liquid) and purified as described in Example 2. The title compound was obtained as a white powder, 21.2 mg.
FAB $(M+H)^+$ at M/Z: 1671.

Amino Acid Analysis: Arg(3.0), Asx(1.0), Cys(1.6), Gly(3.0), Ile(1.9), Phe(2.0), Ser(1.6).

The 300 MHz $^1$H NMR is consistent with the proposed structure.

## Example 23

Seryl-Seryl-Cysteinyl-D-Alanyl-Phenylalanyl-Glycyl-Glycyl-Arginyl-Leucyl-Glycyl-Arginyl-Isoleucyl-Cysteinyl-Phenylalanyl-Arginyl-Carboxamide Cyclic Disulfide

0.56 g protected peptide resin, prepared from p-methylbenzhydrylamine resin and the amino acids added sequentially in the following order: Boc-Arg(N$^G$-Tos), Boc-Phe, Boc-Cys(4-MeBzl), Boc-Ile, Boc-Arg(N$^G$-Tos), Boc-Gly, Boc-Leu, Boc-Arg(N$^G$-Tos), Boc-Gly, Boc-Gly, Boc-Phe, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 19.3 mg.
FAB $(M+H)^+$ at M/Z: 1626.

Amino Acid Analysis: Arg(3.0), Cys(1.6), Gly(3.0), Ile(1.0), Leu(0.9), Phe(1.8), Ser(1.5), Ala(1.0).

The 300 MHz $^1$H NMR is consistent with the proposed structure.

## Example 24

Seryl-Seryl-D-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-D-Cys(4-MeBzl), Boc-Ser(Bzl), Boc-Ser(Bzl), was treated with HF (liquid) and purified as described in Example 2. The title compound was obtained as a white powder, 15 mg.
FAB $(M+H)^+$ at M/Z: 1686.

Amino Acid Analysis: Arg(3.0), Asx(1.0), Cys(1.7), Gly(2.1), Ile(1.9), Phe(1.9), Ser(1.5), Ala(1.0).

The 300 MHz $^1$H NMR is consistent with the proposed structure.

## Example 25

Seryl-Seryl-Cysteinyl-D-Alanyl-Tryptophyl-Glycyl-Glycyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg-($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Trp, Boc-D-Ala, Boc-Cys(4-MeBzl), Boc-Ser(Bzl), Boc-Ser(Bzl), was treated with HF (liquid and purified as described in Example 2. The title compound was obtained as a white powder, 14 mg.
FAB $(M+H)^+$ at M/Z: 1724

Amino Acid Analysis: Arg(3.0), Asx(1.1), Cys(1.9), Gly(2.2), Ile(2.0), Phe(1.1), Ser(1.5), Ala(1.1), Trp(0.8).

The 300 MHz $^1$H NMR is consistent with the proposed structure.

## Example 26

Seryl-Seryl-Cysteinyl-D-Valyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Val, Boc-Cys(4-MeBzl), Boc-Ser(Bzl), Boc-Ser(Bzl), was treated with HF (liquid) and purified as describe din Example 2. The title compound was obtained as a white powder, 30 mg.
FAB $(M+H)^+$ at M/Z: 1715.

Amino Acid Analysis: Arg(2.5), Asx(1.0), Cys(1.5), Gly(2.0), Ile(2.0), Phe(1.8), Ser(1.5), Ala(0.8).

The 300 MHz $^1$H NMR is consistent with the proposed structure.

## Example 27

Seryl-Seryl-Cysteinyl-D-Cyclohexylalanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Cha, Boc-Cys(4-MeBzl), Boc-Ser(Bzl), Boc-Ser(Bzl), was treated with HF (liquid) and purified as described in Example 2. The title compound was obtained as a white powder, 24 mg.
FAB $(M+H)^+$ at M/Z: 1767.

Amino Acid Analysis: Arg(3.0), Asx(1.0), Cys(1.6), Gly(2.1), Ile(2.0), Phe(2.0), Ser(1.8).

The 300 MHz $^1$H NMR is consistent with the proposed structure.

## Example 28

Seryl-Seryl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 19 mg.
FAB $(M+H)^+$ at M/Z: 1685.

Amino Acid Analysis: Arg(3.0), Asx(1.0), Cys(1.8), Gly(2.1), Ile(2.0), Phe(2.1), Ser(1.8), Ala(0.9).

The 300 MHz $^1$H NMR is consistent with the proposed structure.

## Example 29

Seryl-Seryl-Cysteinyl-Glycyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.47 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-Gly, Boc-Cys(4-MeBzl), Boc-Ser(Bzl), Boc-Ser(Bzl), was treated with HF (liquid) and purified as described in Example 2. The title compound was obtained as a white powder, 5.1 mg.
FAB $(M+H)^+$ at M/Z: 1672.

Amino Acid Analysis: Arg(2.3), Asx(1.1), Cys(2.0), Gly(3.4), Ile(2.0), Phe(2.2), Ser(1.7).

The 300 MHz $^1$H NMR is consistent with the proposed structure.

## Example 30

N-AcetylSeryl-Seryl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.44 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-Ser(Bzl), Boc-Ser(Bzl), was acetylated with acetylimidazole and was treated with HF (liquid) and purified as described in Example 2. The title compound was obtained as a white powder, 17.8 mg.
FAB $(M+H)^+$ at M/Z: 1728.

Amino Acid Analysis: Arg(3.0), Asx(1.1), Cys(1.7), Gly(2.2), Ile(2.1), Phe(2.2), Ser(1.7), Ala(1.1).

The 300 MHz $^1$H NMR is consistent with the proposed structure.

## Example 31

Seryl-Seryl-Cysteinyl-D-Alanyl-Tetrahydroisoquinolin-3-oyl-Glycyl-Glycyl-Arginyl-Leucyl-Aspartyl-Arginyl-Isoleucyl-Cysteinyl-Phenylalanyl-Arginyl-Carboxamide Cyclic Disulfide

0.50 g protected peptide resin, prepared from p-methylbenzhydrylamine resin and the amino acids added sequentially in the following order: Boc-Arg($N^G$-Tos), Boc-Phe, Boc-Cys(4-MeBzl), Boc-Ile, Boc-Arg($N^G$-Tos), Boc-Asp($\beta$-cyclohexyl), Boc-Leu, Boc-Arg($N^G$-Tos), Boc-Gly, Boc-Gly, Boc-Tic, Boc-D-Ala, Boc-Cys(4-MeBzl), Boc-Ser(Bzl), Boc-Ser(Bzl), was treated with HF (liquid) and purified as described in Example 2.. The title compound was obtained as a white powder, 17.9 mg.
FAB $(M+H)^+$ at M/Z: 1696.

Amino Acid Analysis: Arg(3.0), Asx(1.1), Cys(1.8), Gly(2.1), Ile(1.0), Leu(1.1), Phe(1.1), Ser(1.5), Ala(1.1).

The 300 MHz $^1$H NMR is consistent with the proposed structure.

Example 32

Arginyl-Cysteinyl-D-Alanyl-Phenylalanyl-Glycyl-Glycyl-Arginyl-D-Leucyl-Aspartyl-Arginyl-Isoleucyl-Cysteinyl-Phenylalanyl-Arginyl-Carboxamide Cyclic Disulfide

0.60 g protected peptide resin, prepared from p-methylbenzhydrylamine resin and the amine acids added sequentially in the following order: Boc-Arg($N^G$-Tos), Boc-Phe, Boc-Cys(4-MeBzl), Boc-Ile, Boc-Arg-($N^G$-Tos), Boc-Asp($\beta$-cyclohexyl), Boc-D-Leu, Boc-Arg($N^G$-Tos), Boc-Gly, Boc-Gly, Boc-Phe, Boc-D-Ala, Boc-Cys(4-MeBzl), Boc-Arg($N^G$-Tos), was treated with HF (liquid) and purified as described in Example 2. The title compound was obtained as a white powder, 36.5 mg.
FAB $(M+H)^+$ at M/Z: 1668.

Amino Acid Analysis: Arg(4.0), Asx(1.2), Cys(1.7), Gly(2.3), Ile(1.1), Leu(1.1), Phe(2.0), Ala(1.0).

The 300 MHz $^1$H NMR is consistent with the proposed structure.

Example 33

8-Aminooctanoyl-Cysteinyl-Beta-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.4 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-$\beta$-Ala, Boc-Cys(4-MeBzl), Boc-Aocta, was treated with HF (liquid) and purified as described in Example 2.. The title compound was obtained as a white powder, 6.0 mg.
FAB $(M+H)^+$ at M/Z: 1652.

Amino Acid Analysis: Arg(3.0), Asx(1.0), Cys(1.9), Gly(2.2), Ile(1.9), Phe(1.9).

The 300 MHz $^1$H NMR is consistent with the proposed structure.

Example 34

Seryl-Seryl-Cysteinyl-D-Alanyl-Phenylalanyl-Glycyl-Glycyl-D-Arginyl-Isoleucyl-Aspartyl-D-Arginyl-Isoleucyl-Cysteinyl-Phenylalanyl-Arginine Cyclic Disulfide

0.46 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-D-Arg($N^G$-Tos), Boc-Asp($\beta$-cyclohexyl), Boc-Ile, Boc-D-Arg($N^G$-Tos), Boc-Gly, Boc-Gly, Boc-Phe, Boc-D-Ala, Boc-Cys(4-MeBzl), Boc-Ser(Bzl), Boc-Ser(Bzl), was treated with HF (liquid and purified as described in Example 2. The title compound was obtained as a white powder, 23 mg.
FAB $(M+H)^+$ at M/Z: 1685.

Amino Acid Analysis: Arg(2.5), Asx(0.8), Cys(1.2), Gly(2.0), Ile(2.0), Phe(2.0), Ser(1.6), Ala(1.1).

The 300 MHz $^1$H NMR is consistent with the proposed structure.

Example 35

Seryl-Seryl-Cysteinyl-D-Alanyl-Phenylalanyl-Glycyl-Glycyl-Arginyl-Isoleucyl-Seryl-Arginyl-Isoleucyl-Cysteinyl-Phenylalanyl-Arginine Cyclic Disulfide

0.40 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Ser(Bzl), Boc-Ile, Boc-Arg($N^G$-Tos), Boc-Gly, Boc-Gly, Boc-Phe, Boc-D-Ala, Boc-Cys(4-MeBzl), Boc-Ser-(Bzl), Boc-Ser(Bzl), was treated with HF (liquid) and purified as described in Example 2. The title compound was obtained as a white powder, 10 mg.
FAB $(M+H)^+$ at M/Z: 1657.

Amino Acid Analysis: Arg(2.5), Cys(1.6), Gly(2.2), Ile(2.0), Phe(2.0), Ser(2.2), Ala(1.2).

The 300 MHz $^1$H NMR is consistent with the proposed structure.

Example 36

Seryl-Seryl-Cysteinyl-2-Aminoisobutyroyl-Phenylalanyl-Glycyl-Glycyl-Arginyl-Leucyl-Aspartyl-Arginyl-Isoleucyl-Cysteinyl-Phenylalanyl-Arginyl-Carboxamide Cyclic Disulfide

0.50 g protected peptide resin, prepared from p-methylbenzhydrylamine resin and the amino acids added sequentially in the following order: Boc-Arg($N^G$-Tos), Boc-Phe, Boc-Cys(4-MeBzl), Boc-Ile, Boc-Arg-($N^G$-Tos), Boc-Asp($\beta$-cyclohexyl), Boc-Leu, Boc-Arg($N^G$-Tos), Boc-Gly, Boc-Gly, Boc-Phe, Boc-Aib, Boc-Cys(4-MeBzl), Boc-Ser(Bzl), Boc-Ser(Bzl), was treated with HF (liquid) and purified as described in Example 2. The title compound was obtained as a white powder, 13.0 mg.
FAB $(M+H)^+$ at M/Z: 1698.
Amino Acid Analysis: Arg(3.0), Asx(1.0), Cys(2.1), Gly(2.2), Ile(1.0), Leu(1.0), Phe(2.0), Ser(1.6).
The 300 MHz $^1$H NMR is consistent with the proposed structure.

Example 37

Seryl-Seryl-Cysteinyl-D-Alanyl-D-Tetrahydroisoquinolin-3-oyl-Glycyl-Glycyl-Arginyl-Isoleucyl-Aspartyl-Arginyl-Isoleucyl-Cysteinyl-Phenylalanyl-Arginyl Cyclic Disulfide

0.50 g protected peptide resin, prepared from N-alpha-Boc-Arg($N^G$-Tosyl) Merrifield resin and the amino acids added sequentially in the following order: Boc-Phe, Boc-Cys(MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-D-Tic, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 10 mg.
FAB $(M+H)^+$ at M/Z: 1698.
Amino Acid Analysis: Arg(3.2), Asx(1.0), Cys(1.8), Gly(2.1), Ile(1.9), Phe(0.9), Ser(1.4), Ala(0.9).
The 300 MHz $^1$H NMR is consistent with the proposed structure.

## Example 38

Seryl-Seryl-Cysteinyl-D-Alanyl-Tyrosyl-Glycyl-Glycyl-Arginyl-Isoleucyl-Aspartyl-Arginyl-Isoleucyl-Cysteinyl-Phenylalanyl-Arginyl Cyclic Disulfide

0.50 g protected peptide resin, prepared from N-alpha-Boc-Arg($N^G$-Tosyl) Merrifield resin and the amino acids added sequentially in the following order: Boc-Phe, Boc-Cys(MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-Tyr(2,6-$Cl_2$Bzl), Boc-D-Ala, Boc-Cys(4-MeBzl), Boc-Ser(Bzl), Boc-Ser(Bzl), was treated with HF (liquid) and purified as described in Example 2. The title compound was obtained as a white powder, 10 mg.
FAB $(M+H)^+$ at M/Z: 1702.
Amino Acid Analysis: Arg(3.2), Asx(1.0), Cys(1.8), Gly(2.1), Ile(1.9), Phe(0.9), Ser(1.4), Ala(1.0), Tyr(0.8).
The 300 MHz $^1$H NMR is consistent with the proposed structure.

## Example 39

Seryl-Seryl-Cysteinyl-D-Alanyl-N-Methylphenylalanyl-Glycyl-Glycyl-Arginyl-Isoleucyl-Aspartyl-Arginyl-Isoleucyl-Cysteinyl-Phenylalanyl-Arginyl Cyclic Disulfide

0.50 g protected peptide resin, prepared from N-alpha-Boc-Arg($N^G$-Tos) Merrifield resin and the amino acids added sequentially in the following order: Boc-Phe, Boc-Cys(MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-D-Ala-N-MePhe, Boc-Cys(4-MeBzl), Boc-Ser(Bzl), Boc-Ser(Bzl), was treated with HF (liquid) and purified as described in Example 2. The title compound was obtained as a white powder, 10 mg.
FAB $(M+H)^+$ at M/Z: 1700.
Amino Acid Analysis: Arg (3.2), Asx(1.0), Cys(1.8), Gly(2.1), Ile(1.9), Phe(0.9), Ser(1.4), Ala(0.9).
The 300 MHz $^1$H NMR is consistent with the proposed structure.

## Example 40

Seryl-Seryl-Cysteinyl-D-Alanyl-Phenylalanyl-Glycyl-Glycyl-Arginyl-Isoleucyl-Aspartyl-Alanyl-Isoleucyl-Cysteinyl-Phenylalanyl-Arginyl Cyclic Disulfide

0.50 g protected peptide resin, prepared from N-alpha-Boc-Arg($N^G$-Tosyl) Merrrifield resin and the amino acids added sequentially in the following order: Boc-Phe, Boc-Cys(MeBzl), Boc-Ile, Boc-Ala, Boc-Asp(β-cyclohexyl), Boc-Ile, Boc-Arg($N^G$-Tos), Boc-Gly, Boc-Gly, Boc-Phe, Boc-D-Ala, Boc-Cys(4-MeBzl), Boc-Ser(Bzl), Boc-Ser(Bzl) was treated with HF (liquid) and purified as described in Example 2. The title compound was obtained as a white powder, 10 mg.
FAB $(M+H)^+$ at M/Z: 1600.
Amino Acid Analysis: Arg(2.2), Asx(1.0), Cys(1.8), Gly(2.1), Ile(1.9), Ser(1.4), Ala(1.9).
The 300 MHz $^1$H NMR is consistent with the proposed structure.

## Example 41

Seryl-Seryl-Cysteinyl-D-Alanyl-Phenylalanyl-Glycyl-Glycyl-Citrullinyl-Isoleucyl-Aspartyl-Arginyl-Isoleucyl-Cysteinyl -Phenylalanyl-Arginyl Cyclic Disulfide

0.50 g protected peptide resin, prepared from N-alpha-Boc-Arg($N^G$-Tos) Merrifield resin and the amino acids added sequentially in the following order: Boc-Phe, Boc-Cys(MeBzl), Boc-Ile, Boc-Arg($N^G$-Tos), Boc-Asp(β-cyclohexyl), Boc-Ile, Boc-Cit, Boc-Gly, Boc-Gly, Boc-Phe, Boc-D-Ala, Boc-Cys(4-MeBzl), Boc-Ser-(Bzl), Boc-Ser(Bzl) was treated with HF (liquid) and purified as described in Example 2. The title compound was obtained as a white powder, 10 mg.
FAB $(M+H)^+$ at M/Z: 1686.
Amino Acid Analysis: Arg(2.2), Asx(1.0), Cys(1.8), Gly(2.1), Ile(1.9), Phe(1.9), Ser(1.4), Ala(0.9).
the 300 MHz $^1$H NMR is consistent with the proposed structure.

## Example 42

Seryl-Seryl-Cysteinyl-D-Alanyl-Phenylalanyl-4-Aminophenylacetyl-Arginyl-Isoleucyl-Aspartyl-Arginyl-Isoleucyl-Cysteinyl-Phenylalanyl-Arginyl Cyclic Disulfide.

0.45 g protected peptide resin, prepared from N-alpha-t-Boc-Arg($N^G$-Tos) Merrifield resin and the amino acids added sequentially in the following order: Boc-Phe, Boc-Cys(MeBzl), Boc-Ile, Boc-Arg($N^G$-Tos), Boc-Asp(β-cyclohexyl), Boc-Ile, Boc-Arg($N^G$-Tos), Boc-Phe-Papaa, Boc-D-Ala, Boc-Cys(4-MeBzl), Boc-Ser(Bzl), Boc-Ser(Bzl) was treated with HF (liquid) and purified as described in Example 2. The title compound was obtained as a white powder, 19 mg.
FAB $(M+H)^+$ at M/Z: 1704.
Amino Acid Analysis: Ala(1.0), Arg(3.0), Asx(1.2), Cys(1.4), Ile(1.8), Phe(2.0), Ser(1.6).
The 300 MHz $^1$H NMR is consistent with the proposed structure.

## Example 43

Seryl-Seryl-Cysteinyl-D-Alanyl-Phenylalanyl-3-Aminophenylacetyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Phe-Mapaa, Boc-D-Ala, Boc-Cys(4-MeBzl), Boc-Ser(Bzl), Boc-Ser(Bzl) was treated with HF (liquid) and purified as described in Example 2. The title compound was obtained as a white powder, 12 mg.
FAB $(M+H)^+$ at M/Z: 1704.
Amino Acid Analysis: Ala(1.0), Arg(3.0), Asx(1.1), Cys(1.5), Ile(1.9), Phe(2.0), Ser(1.7).
the 300 MHz $^1$H NMR is consistent with the proposed structure.

Example 44

Seryl-Seryl-Cysteinyl-Phenylalanyl-Phenylalanyl-4-Aminophenylacetyl-Arginyl-Isoleucyl-Aspartyl-Arginyl-Isoleucyl-Cysteinyl-Phenylalanyl-Arginyl Cyclic Disulfide

0.46 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Phe-Papaa, Boc-Phe, Boc-Cys(4-MeBzl), Boc-Ser(Bzl), Boc-Ser(Bzl) was treated with HF (liquid) and purified as described in Example 2. The title compound was obtained as a white powder, 6 mg.

FAB $(M+H)^+$ at M/Z: 1781.

Amino Acid Analysis: Arg(3.0), Asx(1.2), Cys(1.3), Ile(2.0), Phe(2.0), Ser(1.7).

The 300 MHz $^1$H NMR is consistent with the proposed structure.

Example 45

8-Aminooctanoyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.47 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-Aocta, was treated with HF (liquid) and purified as described in the previous example. The title compound was obtained as a white powder, 39.4 mg.

FAB $(M+H)^+$ at M/Z: 1653.

Amino Acid Analysis: Asx(1.0), Gly(2.1), Ala(1.1), Cys(1.9), Ile(1.9), Phe(1.9), Arg(3.0).

The 300 MHz $^1$H NMR is consistent with the proposed structure.

Example 46

6-Aminohexanoyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-Ahexa, was treated with HF (liquid) and purified as described in Example 2. The title compound was obtained as a white powder, 35.5 mg.

FAB $(M+H)^+$ at M/Z: 1625.

Amino Acid Analysis: Asp(0.9), Gly(2.0), Ala(1.0), Cys(1.6), Ile(2.0), Phe(1.9), Arg(3.0).

The 300 MHz $^1$H NMR is consistent with the proposed structure.

Example 47

11-Aminoundecanoyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.50 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-Phe, Boc-D-Ala, Boc-Cys(4-MeBzl), Boc-Aunda, was treated with HF (liquid) and purified as described in Example 2. The title compound was obtained as a white powder, 18.5 mg.
FAB $(M+H)^+$ at M/Z: 1695.
Amino Acid Analysis: Asx(1.0), Gly(2.1), Ala(1.0), Cys(1.6), Ile(2.1), Phe(2.3), Arg(3.0).
The 300 MHz $^1$H NMR is consistent with the proposed structure.

## Example 48

5-Aminopentanoyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.47 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-Phe, Boc-D-Ala, Boc-Cys(4-MeBzl), Boc-Apenta, was treated with HF (liquid) and purified as described in Example 2. The title compound was obtained as a white powder, 17.1 mg.
FAB $(M+H)^+$ at M/Z: 1610.
Amino Acid Analysis: Asx(1.1), Gly(2.2), Ala(1.1), Cys(1.6), Ile(2.1), Phe(2.2), Arg(3.0).
The 300 MHz $^1$H NMR is consistent with the proposed structure.

## Example 49

3-((S)-Hydroxymethyl)-3-Aminopropanoyl-Cysteinyl-(D)-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-Phe, Boc-D-Ala, Boc-Cys(4-MeBzl), Boc-HSer, was treated with HF (liquid) and purified as described in Example 2. The title compound was obtained as a white powder, 21.9 mg.
FAB $(M+H)^+$ at M/Z: 1614.
Amino Acid Analysis: Asx(0.9), Gly(1.9), Ala(0.9), Cys(1.3), Ile(2.1), Phe(2.3), Arg(3.0).
The 300 MHz $^1$H NMR is consistent with the proposed structure.

## Example 50

Seryl-Lysyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.46 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-Phe, Boc-D-Ala, Boc-Cys(4-MeBzl), Boc-Lys(2-Cl-Z), Boc-Ser(Bzl), was treated with HF (liquid) and purified as described in Example 2. The title compound was obtained as a white powder, 28.2 mg.
FAB $(M+H)^+$ at M/Z: 1726.
Amino Acid Analysis: Asx(1.1), Ser(0.9), Gly(2.0), Ala(1.0), Cys(1.8), Ile(2.0), Phe(2.1), Lys(0.9), Arg(3.0).

The 300 MHz ¹H NMR is consistent with the proposed structure.

## Example 51

Seryl-Seryl-Cysteinyl-D-Alanyl-Phenylalanyl-D-Alanyl-Glycyl-Arginyl-Isoleucyl-Aspartyl-Arginyl-Isoleucyl-Cysteinyl-Phenylalanyl-Arginyl Cyclic Disulfide

0.45 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-D-Ala, Boc-Phe, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 13.3 mg.
FAB $(M+H)^+$ at M/Z: 1699.
Amino Acid Analysis: Asx(1.0), Ser(1.5), Gly(1.1), Ala(2.1), Cys(1.9), Ile(2.5), Phe(2.0), Arg(3.0).
The 300 MHz ¹H NMR is consistent with the proposed structure.

## Example 52

Lysyl-Cysteinyl-D-Valyl-Phenylalanyl-Glycyl-Glycyl-Arginyl-Isoleucyl-Aspartyl-Arginyl-Isoleucyl-Cysteinyl-Cyclohexylalanyl-Arginyl Cyclic Disulfide

0.45 g protected peptide resin, prepared from N-alpha-t-Boc-Arg($N^G$-Tos) Merrifield resin and the amino acids added sequentially in the following order: Boc-Cha, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-Phe, Boc-D-Val, Boc-Cys(4-MeBzl), Boc-Lys(2-Cl-Z), was treated with HF (liquid) and purified as described in Example 2. The title compound was obtained as a white powder, 15.2 mg.
FAB $(M+H)^+$ at M/Z: 1675.
Amino Acid Analysis: Asx(0.9), Gly(1.8), Cys(1.4), Val(0,9), Ile(2.1), Phe(1.1), Lys(0.9), Arg(3.1).
The 300 MHz ¹H NMR is consistent with the proposed structure.

## Example 53

Seryl-Seryl-Cysteinyl-D-Alanyl-Phenylalanyl-Alanyl-Glycyl-Arginyl-Isoleucyl-Aspartyl-Arginyl-Isoleucyl-Cysteinyl-Phenylalanyl-Arginyl Cyclic Disulfide

0.50 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-Ala, Boc-Phe, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 36.0 mg.
FAB $(M+H)^+$ at M/Z: 1700.
Amino Acid Analysis: Asx(1.0), Ser(1.4), Gly(1.1), Ala(1.9), Cys(1.5), Ile(2.1), Phe(2.0), Arg(3.3).
The 300 MHz ¹H NMR is consistent with the proposed structure.

## Example 54

Seryl-Seryl-Cysteinyl-D-Alanyl-(3,4-dimethoxy)-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.62 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-DimeDOPA, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 27.2 mg.
FAB $(M+H)^+$ at M/Z: 1745.
Amino Acid Analysis: Asx(0.9), Ser(1.4), Gly(1.9), Ala(1,0), Cys(1.6), Ile(2.0), Phe(1.1), Arg(3.1).
The 300 MHz $^1$H NMR is consistent with the proposed structure.

## Example 55

Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.88 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), was treated with HF (liquid) and purified as described in the previous example. The title compound was obtained as a white powder, 63.0 mg.
FAB $(M+H)^+$ at M/Z: 1511.
Amino Acid Analysis: Asx(1.0), Gly(1.9), Ala(1.0), Cys(1.7), Ile(2.0), Phe(2.1), Arg(3.2).
The 300 MHz $^1$H NMR is consistent with the proposed structure.

## Example 56

Arginyl-Arginyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.82 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-Arg($N^G$-Tos), Boc-Arg($N^G$-Tos), was treated with HF (liquid) and purified as described in Example 2. The title compound was obtained as a white powder, 39.7 mg.
FAB $(M+H)^+$ at M/Z: 1824.
Amino Acid Analysis: Asx(1.0), Gly(2.0), Ala(1.0), Cys(1.8), Ile(1.9), Phe(2.0), Arg(5.1).
The 300 MHz $^1$H NMR is consistent with the proposed structure.

## Example 57

Seryl-Seryl-Cysteinyl-D-Alanyl-(3,4-Dihydroxy)phenylalanyl-Glycyl-Glycyl-Arginyl-Isoleucyl-Aspartyl-Arginyl-Isoleucyl-Cysteinyl-Phenylalanyl-Arginyl Cyclic Disulfide

1.23 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-DOPA, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 3.7 mg.
FAB $(M+H)^+$ at M/Z: 1718.
Amino Acid Analysis: Asx(1.0), Ser(1.5), Gly(1.9), Ala(1.0), Cys(1.8), Ile(2.0), Phe(1.0), Arg(3.2).
The 300 MHz $^1$H NMR is consistent with the proposed structure.

## Example 58

4-Aminobutanoyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.50 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-Abuta, was treated with HF (liquid) and purified as described in Example 2. The title compound was obtained as a white powder, 38.1 mg.
FAB $(M+H)^+$ at M/Z: 1596.
Amino Acid Analysis: Asx(1.0), Gly(1.9), Ala(0.9), Cys(1.7), Ile(1.9), Phe(1.9), Arg(3.3).
The 300 MHz $^1$H NMR is consistent with the proposed structure.

## Example 59

N-Acetyl-5-Aminopentanoyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.26 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Acetyl-apenta, was treated with HF (liquid) and purified as described in example 2. The title compound was obtained as a white powder, 12.2 mg.
FAB $(M+H)^+$ at M/Z: 1653.
Amino Acid Analysis: Asx(1.0), Gly(2.1), Ala(1.1), Cys(1.8), Ile(1,9), Phe(2.0), Arg(3.3).
The 300 MHz $^1$H NMR is consistent with the proposed structure.

## Example 60

Arginyl-Cysteinyl-D-Alanyl-Phenylalanyl-Glycyl-Glycyl-Arginyl-Isoleucyl-Aspartyl-Arginyl-Isoleucyl-Cysteinyl-Cyclohexylalanyl-Arginyl Cyclic Disulfide

0.76 g protected peptide resin, prepared from N-alpha-t-Boc-Arg($N^G$-Tos) Merrifield resin and the amino acids added sequentially in the following order: Boc-Cha, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-Arg($N^G$-Tos), was treated with HF (liquid) and purified as described in Example 2. The title compound was obtained as a white powder, 34.0 mg.
FAB $(M+H)^+$ at M/Z: 1674.
Amino Acid Analysis: Asx(1.1), Gly(2.0), Ala(1.0), Cys(1.9), Ile(2.0), Phe(1.1), Arg(3.9).
The 300 MHz $^1$H NMR is consistent with the proposed structure.

## Example 61

Seryl-Seryl-Cysteinyl-D-Alanyl-Phenylalanyl-Glycyl-Alanyl-Arginyl-Isoleucyl-Aspartyl-Arginyl-Isoleucyl-Cysteinyl-Phenylalanyl-Arginyl Cyclic Disulfide

0.50 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Ala, Boc-Gly, Boc-Phe, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 30.0 mg.
FAB $(M+H)^+$ at M/Z: 1699.
Amino Acid Analysis: Arg(3.0), Asx(0.9), Cys(1.5), Gly(1.0), Ile(2.0), Phe(1.9), Ser(1.2), Ala(1.8).
The 300 MHz $^1$H NMR is consistent with the proposed structure.

Example 62

Seryl-Seryl-Cysteinyl-D-Alanyl-Phenylalanyl-trans-4-Aminomethylcyclohexan-1-oyl-Arginyl-Leucyl-Aspartyl-Arginyl-Isoleucyl-Cysteinyl-Phenylalanyl-Arginyl Carboxamide Cyclic Disulfide

0.50 g protected peptide resin, prepared from p-methylbenzhydrylamine resin and the amino acids added sequentially in the following order: Boc-Arg($N^G$-Tos), Boc-Phe, Boc-Cys(4-MeBzl), Boc-Ile, Boc-Arg($N^G$-Tos), Boc-Asp(β-cyclohexyl), Boc-Leu, Boc-Arg($N^G$-Tos) Boc-AMCCA, Boc-Phe, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 9.8 mg.
FAB $(M+H)^+$ at M/Z: 1712.
Amino Acid Analysis: Arg(3.1), Asx(1.0), Ala(1.1), Ile(1.0), Leu(1.1), Phe(1.9), Ser(2.2).
The 300 MHz $^1$H NMR is consistent with the proposed structure.

Example 63

Seryl-Seryl-Cysteinyl-D-Alanyl-Tetrahydroisoquinolin-3-oyl-Glycyl-Glycyl-Arginyl-Leucyl-Aspartyl-Arginyl-Isoleucyl-Cysteinyl-Arginyl Carboxamide Cyclic Disulfide

0.50 g protected peptide resin, prepared from p-methylbenzhydrylamine resin and the amino acids added sequentially in the following order: Boc-Arg($N^G$-Tos), Boc-Cys(4-MeBzl), Boc-Ile, Boc-Arg($N^G$-Tos), Boc-Asp(β-cyclohexyl), Boc-Leu, Boc-Arg($N^G$-Tos), Boc-Gly, Boc-Gly, Boc-Tic, Boc-D-Ala, Boc-Cys(4-MeBzl), Boc-Ser(Bzl), Boc-Ser(Bzl), was treated with HF (liquid) and purified as described in Example 2. The title compound was obtained as a white powder. 7.0 mg.
FAB $(M+H)^+$ at M/Z: 1550.
Amino Acid Analysis: Arg(3.1), Asx(1.0), Cys(1.5), Gly(1.9), Ile(1.0), Leu(1.1), Ser(1.7), Ala(1.0).
The 300 MHz $^1$H NMR is consistent with the proposed structure.

Example 64

Arginyl-Cysteinyl-D-Alanyl-Phenylalanyl-Glycyl-Glycyl-Arginyl-Leucyl-Aspartyl-Arginyl-Isoleucyl-Cysteinyl-D-Alanyl-Phenylalanyl-Arginyl Carboxamide Cyclic Disulfide

0.50 g protected peptide resin, prepared from p-methylbenzhydrylamine resin and the amino acids added sequentially in the following order: Boc-Arg($N^G$-Tos), Boc-Phe, Boc-D-Ala, Boc-Cys(4-MeBzl), Boc-Ile, Boc-Arg($N^G$-Tos), Boc-Asp($\beta$-cyclohexyl), Boc-Leu, Boc-Arg($N^G$-Tos) Boc-Gly, Boc-Gly, Boc-Phe, Boc-D-Ala, Boc-Cys(4-MeBzl), Boc-Arg($N^G$-Tos), was treated with HF (liquid) and purified as described in Example 2. The title compound was obtained as a white powder, 39.0 mg.
FAB $(M+H)^+$ at M/Z: 1738.
Amino Acid Analysis: Arg(4.0), Asx(1.0), Cys(1.8), Gly(2.1), Ile(1.1), Leu(1.0), Phe(2.0), Ala(1.9).
The 300 MHz $^1$H NMR is consistent with the proposed structure.

## Example 65

Arginyl-Cysteinyl-D-Alanyl-Phenylalanyl-Glycyl-Glycyl-Arginyl-Leucyl-Aspartyl-Arginyl-Leucyl-Cysteinyl-Phenylalanyl-Arginyl Carboxamide Cyclic Disulfide

0.50 g protected peptide resin, prepared from p-methylbenzhydrylamine resin and the amino acids added sequentially in the following order: Boc-Arg($N^G$-Tos), Boc-Phe, Boc-Cys(4-MeBzl), Boc-Leu, Boc-Arg($N^G$-Tos), Boc-Asp($\beta$-cyclohexyl), Boc-Leu, Boc-Arg($N^G$-Tos), Boc-Gly, Boc-Gly, Boc-Phe, Boc-D-Ala, Boc-Cys(4-MeBzl), Boc-Arg($N^G$-Tos), was treated with HF (liquid) and purified as described in Example 2. The title compound was obtained as a white powder, 44.0 mg.
FAB $(M+H)^+$ at M/Z: 1669.
Amino Acid Analysis: Arg(3.8), Asx(1.0), Cys(2.1), Gly(2.1), Leu(2.0), Phe(1.9), Ala(1.3).
The 300 MHz $^1$H NMR is consistent with the proposed structure.

## Example 66

Seryl-Seryl-Cysteinyl-D-Alanyl-Phenylalanyl-Glycyl-Glycyl-Arginyl-Isoleucyl-Aspartyl-Arginyl-Isoleucyl-Cysteinyl-Cysteinyl-2-Thienylalanyl-Arginyl Cyclic Disulfide

0.89 g protected peptide resin, prepared from N-alpha-t-Boc-Arg($N^G$-Tos) Merrifield resin and the amino acids added sequentially in the following order: Boc-2-Thi, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 5 mg.
FAB $(M+H)^+$ at M/Z: 1692.
Amino Acid Analysis: Ala(1.1), Arg(2.8), Asx(1.1), Gly(2.2), Ile(1.9), Phe(0.8), Ser(1.2).
The 300 MHz $^1$H NMR is consistent with the proposed structure.

## Example 67

Seryl-Seryl-Cysteinyl-Glycyl-Phenylalanyl-Glycyl-Glycyl-Arginyl-Isoleucyl-Aspartyl-Arginyl-Isoleucyl-Cysteinyl-Phenylalanyl-Glycyl Cyclic Disulfide

0.96 g protected peptide resin, prepared from N-alpha-t-Boc-Gly Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-Gly, Boc-Cys(4-MeBzl), Boc-Ser(Bzl), Boc-Ser(Bzl), was treated with HF (liquid) and purified as described in Example 2. The title compound was obtained as a white powder, 30 mg.
FAB $(M+H)^+$ at M/Z: 1572.
Amino Acid Analysis: Arg(2.0), Asx(0.9), Cys(1.3), Gly(4.1), Ile(1.8), Phe(2.0), Ser(1.5).
The 300 MHz $^1$H NMR is consistent with the proposed structure.

## Example 68

### Seryl-Seryl-Cysteinyl-Glycyl-Phenylalanyl-Glycyl-Glycyl-Arginyl-Isoleucyl-Aspartyl-Arginyl-Isoleucyl-Cysteinyl-Phenylalanyl-Lysyl Cyclic Disulfide

1.46 g protected peptide resin, prepared from N-alpha-t-Boc-Lys(2-Cl-Z) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-Gly, Boc-Cys(4-MeBzl), Boc-Ser(Bzl), Boc-Ser(Bzl), was treated with HF (liquid) and purified as described in Example 2. The title compound was obtained as a white powder, 22 mg.
FAB $(M+H)^+$ at M/Z: 1644.
Amino Acid Analysis: Arg(2.0), Asx(1.1), Gly(3.2), Ile(1.9), Lys (0.9), Phe(1.9), Ser(1.8).
The 300 MHz NMR is consistent with the proposed structure.

## Example 69

### Seryl-Seryl-Cysteinyl-Sarcosinyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

1.25 g protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-Sar, Boc-Cys(4-MeBzl), Boc-Ser(Bzl), Boc-Ser(Bzl), was treated with HF (liquid) and purified as described in Example 2. The title compound was obtained as a white powder, 20 mg.
FAB $(M+H)^+$ at M/Z: 1685.
Amino Acid Analysis: Arg(2.8), Asx(1.1), Cys(1.1), Gly(2.3), Ile(2.0), Phe(2.0), Ser(1.6).
The 300 MHz NMR is consistent with the proposed structure.

## Example 70

### Seryl-Seryl-Cysteinyl-(D)-Alanyl-Phenylalanyl-Glycyl-Glycyl-Arginyl-Isoleucyl-Aspartyl-Arginyl-Isoleucyl-Cysteinyl-1-Aminoindan-oyl-Arginyl Cyclic Disulfide

0.82 g protected peptide resin, prepared from N-alpha-t-Boc-Arg-($N^G$-Tos) Merrifield resin and the amino acids added sequentially in the following order: Boc-Aic, Boc-Cys(4-MeBzl), Boc-Ile, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 55.4 mg.
FAB $(M+H)^+$ at M/Z: 1700.
Amino Acid Analysis: Ala(0.9), Arg(3.0), Asx(1.0), Cys(1.3), Gly(2.0), Ile(1.8), Phe(1.1), Ser(1.5).
The 300 MHz $^1$H NMR is consistent with the proposed structure.

## Example 71

### Seryl-Seryl-Cysteinyl-D-Alanyl-Phenylalanyl-Glycyl-Glycyl-Arginyl-Isoleucyl-Aspartyl-Arginyl-Isoleucyl-Cysteinyl-trans-4-Phenylprolyl-Arginyl Cyclic Disulfide

0.74 g protected peptide resin, prepared from N-alpha-t-Boc-Arg-($N^G$-Tos) Merrifield resin and the amino acids added sequentially in the following order: Boc-trans-4-FPro, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-Phe, Boc-D-Ala, Boc-Cys(4-MeBzl), Boc-Ser(Bzl), Boc-Ser(Bzl), was treated with HF (liquid) and purified as described in Example 2. The title compound was obtained as a white powder, 19.1 mg.
FAB $(M+H)^+$ at M/Z: 1712.
Amino Acid Analysis: Ala(0.8), Arg(3.2), Asx(1.1), Cys(1.3), Gly(1.9), Ile(2.2), Phe(0.9), Ser(1.4).
The 300 MHz $^1$H NMR is consistent with the proposed structure.

## Examples 72 A,B

Seryl-Seryl-Cysteinyl-D-Alanyl-Phenylalanyl-Glycyl-Glycyl-Arginyl-Isoleucyl-beta-Methylaspartyl-Arginyl-Isoleucyl-Cysteinyl Cyclic Disulfide (A) and Seryl-Seryl-Cysteinyl-D-Alanyl-Phenylalanyl-Glycyl-Glycyl-Arginyl-Isoleucyl-Cycloaspartyl-Arginyl-Isoleucyl-Cysteinyl Cyclic Disulfide (B)

1.82 g protected peptide resin, prepared from N-alpha-t-Boc-Cys(4-MeBzl) Merrifield resin and the amino acids added sequentially in the following order: Boc-Ile, Boc-Arg($N^G$-Tos), Boc-Asp(β-methyl), Boc-Ile, Boc-Arg($N^G$-Tos), Boc-Gly, Boc-Gly, Boc-Phe, Boc-D-Ala, Boc-Cys(4-MeBzl), Boc-Ser(Bzl), Boc-Ser-(Bzl),was treated with HF (liquid) and purified as described in Example 2. The title compounds were obtained as white powders, 21.3 mg and 32.4 mg, respectively.
A:
FAB $(M+H)^+$ at M/Z: 1396.
Amino Acid Analysis: Ala(1.0), Arg(2.1), Asx(1.0), Cys(1.6), Gly(2.0), Ile(2.0), Phe(0.9), Ser(1.5).
The 300 MHz $^1$H NMR is consistent with the proposed structure.
B:
FAB $(M+H)^+$ at M/Z: 1364.
Amino Acid Analysis: Ala(1.0), Arg(2.1), Asx(1.0), Cys(1.6), Gly(1.8), Ile(1.9), Phe(1.0), Ser(1.5).
The 300 MHz $^1$H NMR is consistent with the proposed structure.

## Example 73

Seryl-Seryl-Cysteinyl-Glycyl-Phenylalanyl-Glycyl-Glycyl-Arginyl-Isoleucyl-Aspartyl-Arginyl-Isoleucyl-Cysteinyl-beta-Naphthylalanyl-Arginyl Cyclic Disulfide

0.70 g protected peptide resin, prepared from N-alpha-t-Boc-Arg($N^G$-Tos) Merrifield resin and the amino acids added sequentially in the following order: Boc-β-NAl, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-Phe, Boc-Gly, Boc-Cys(4-MeBzl), Boc-Ser(Bzl), Boc-Ser(Bzl), was treated with HF (liquid) and purified as described in Example 2. The title compound, the first of two isomers to elute from the HPLC column, was obtained as a white powder, 8.8 mg.
FAB $(M+H)^+$ at M/Z: 1722.
Amino Acid Analysis: Arg(3.1), Asx(0.9), Cys(1.3), Gly(2.9), Ile(1.9), Phe(1.1, Ser(1.5).
The 300 MHz $^1$H NMR is consistent with the proposed structure.

## Example 74

Seryl-Seryl-Cysteinyl-Glycyl-Phenylalanyl-Glycyl-Glycyl-Arginyl-Isoleucyl-Aspartyl-Arginyl-Isoleucyl-Cysteinyl-beta-Naphthylalanyl-Arginyl Cyclic Disulfide

This compound, the later of two isomers eluting from the HPLC purification described above, is obtained as a white powder, 13.2 mg.
FAB $(M+H)^+$ at M/Z: 1722.
Amino Acid Analysis: Arg(3.1), Asx(0.9), Cys(1.6), Gly(3.0), Ile(2.0), Phe(1.1, Ser(1.5).
The 300 MHz $^1$H NMR is consistent with the proposed structure.

## Example 75

8-Aminooctanoyl-Cysteinyl-D-Alanyl-Phenylalanyl-Glycyl-Glycyl-Arginyl-Isoleucyl-Aspartyl-Arginyl-Isoleucyl-Cysteinyl-Cyclohexylalanyl-Arginyl Cyclic Disulfide

0.50 g protected peptide resin, prepared from N-alpha-t-Boc-Arg-($N^G$-Tos) Merrifield resin and the amino acids added sequentially in the following order: Boc-Cha, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-Phe, Boc-D-Ala, Boc-Cys(4-MeBzl), Boc-Aocta, was treated with HF (liquid) and purified as described in Example 2. The title compound was obtained as a white powder, 15 mg.
FAB $(M+H)^+$ at M/Z: 1658.
Amino Acid Analysis: Arg(3.0), Asx(1.1), Cys(1.0), Gly(2.0), Ile(2.0), Phe(1.1), Ala(1.1).
The 300 MHz $^1$H NMR is consistent with the proposed structure.

## Example 76

Seryl-Seryl-Cysteinyl-D-Alanyl-Phenylalanyl-Glycyl-Glycyl-Arginyl-Isoleucyl-Asparaginyl-Arginyl-Isoleucyl-Cysteinyl-Phenylalanyl-Arginyl Cyclic Disulfide

0.50 g of protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asn, Boc-Ile, Boc-Arg-($N^G$-Tos), Boc-Gly, Boc-Gly, Boc-Phe, Boc-D-Ala, Boc-Cys(4-MeBzl), Boc-Ser(Bzl), Boc-Ser(Bzl) was treated with HF (liquid) and purified as described in Example 2. The title compound was obtained as a white powder, 25 mg. FAB $(M+H)^+$ at M/Z: 1684. Amino Acid Analysis: Arg (3.2), Asx (1.0), Cys (1.8), Gly (2.1), Ile (1.9), Phe (1.9), Ser (1.4), Ala (0.9).
The 300 MHz $^1$H NMR is consistent with the proposed structure.

## Example 77

Seryl-Seryl-Cysteinyl-D-Alanyl-2-Thienylalanyl-Glycyl-Glycyl-Arginyl-Isoleucyl-Aspartyl-Arginyl-Isoleucyl-Cysteinyl-Phenylalanyl-Arginyl Cyclic Disulfide

1.32 g of protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(β- cyclohexyl), Boc-Ile, Boc-Arg($N^G$-Tos), Boc-Gly, Boc-Gly, Boc-2-Thi, Boc-D-Ala, Boc-Cys-(4-MeBzl), Boc-Ser(Bzl), Boc-Ser(Bzl) was treated with HF (liquid) and purified as described in Example 2. The title compound, the first of two isomers to elute from the HPLC column, was obtained as a white powder, 12 mg.
FAB $(M+H)^+$ at M/Z: 1692. Amino Acid Analysis: Ala (1.0), Arg (2.8), Asx (1.0), Cys (1.4), Gly (2.3), Ile (2.0), Phe (1.1), Ser (1.6).
The 300 MHz $^1$H NMR is consistent with the proposed structure.

## Example 78

Seryl-Seryl-Cysteinyl-D-Alanyl-2-Thienylalanyl-Glycyl-Glycyl-Arginyl-Isoleucyl-Aspartyl-Arginyl-Isoleucyl-Cysteinyl-Phenylalanyl-Arginyl Cyclic Disulfide

This compound , the later of two isomers eluting from the HPLC purification described in Example 79, was obtained as a white powder, 12 mg.
FAB $(M+H)^+$ at M/Z: 1692. Amino Acid Analysis: Ala (0.7), Arg (3.1), Asx (1.1), Cys (0.9), Gly (1.7), Ile (2.3), Phe (1.3), Ser (1.8).
The 500 MHz $^1$H NMR is consistent with the proposed structure.

## Example 79

Seryl-Seryl-Cysteinyl-D-Alanyl-Phenylalanyl-Glycyl-Glycyl-Arginyl-Isoleucyl-Aspartyl-Arginyl-Phenylalanyl-Cysteinyl-Isoleucyl-Arginyl Cyclic Disulfide

1.10 g of protected peptide resin, prepared from N-alpha-t-Boc-Arg($N^G$-Tos) Merrifield resin and the amino acids added sequentially in the following order: Boc-Ile, Boc-Cys(4-MeBzl), Boc-Phe, Boc-Arg($N^G$-Tos), Boc-Asp($\beta$-cyclohexyl), Boc-Ile, Boc-Arg($N^G$-Tos), Boc-Gly, Boc-Gly, Boc-Phe, Boc-D-Ala, Boc-Cys(4-MeBzl), Boc-Ser(Bzl), Boc-Ser(Bzl) was treated with HF (liquid) and purified as described in Example 2. The title compound was obtained as a white powder, 35 mg.
FAB $(M+H)^+$ at M/Z: 1686. Amino Acid Analysis: Ala (1.0), Arg (3.1), Asx (1.1), Cys (1.5), Gly (2.0), Ile (2.0), Phe (1.9), Ser (1.2).
The 500 MHz $^1$H NMR is consistent with the proposed structure.

## EXAMPLE 80

Ornithyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.99 g Protected peptide resin, prepared from N-a-t-Boc-Arg-(Ng-Tos) Merrifield resin and the amino acids added sequentially in the following order: Boc-Phe, Boc-Cys(4-MeBzl), Boc-Ile, Boc-Arg(Ng-Tos), Boc-Asp($\beta$-cyclohexyl), Boc-Ile, Boc-Arg(Ng-Tos), Boc-Gly, Boc-Gly, Boc-Phe, Boc-D-Ala, Boc-Cys(4-MeBzl), Boc-Orn(Cbz), was treated with HF (liquid) and purified as described in EXAMPLE 2. The title compound was obtained as a white powder, 62.2 mg.
FAB $(M+H)^+$ at M/Z: 1626
AMINO ACID ANALYSIS: Arg(2.9), Asx(1.0), Cys(1.8), Gly(2.1), Ile(2.0), Phe(2.0), Ala(1.0), Orn(1.0).
The 500 MHz NMR is consistent with the proposed structure.

## EXAMPLE 81

D-Ornithyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.81 g Protected peptide resin, prepared from N-a-t-Boc-Arg-(Ng-Tos) Merrifield resin and the amino acids added sequentially in the following order: Boc-Phe, Boc-Cys(4-MeBzl), Boc-Ile, Boc-Arg(Ng-Tos), Boc-Asp($\beta$-cyclohexyl), Boc-Ile, Boc-Arg(Ng-Tos), Boc-Gly, Boc-Gly, Boc-Phe, Boc-D-Ala, Boc-Cys(4-MeBzl), Boc-Orn(Cbz), was treated with HF (liquid) and purified as described in EXAMPLE 2. The title compound was obtained as a white powder, 46.0 mg.
FAB $(M+H)^+$ at M/Z: 1626
AMINO ACID ANALYSIS: Arg(2.9), Asx(1.0), Cys(1.7), Gly(2.0), Ile(2.0), Phe(2.1), Ala(1.0), Orn(0.9).
The 300 MHz NMR is consistent with the proposed structure.

EXAMPLE 82

Citrullinyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.74 g Protected peptide resin, prepared from N-alpha-t-Boc-Arg-($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Boc-Gly, Boc-Phe, Boc-D-Ala, Boc-Cys(4-MeBzl), Boc-Cit, was treated with HF (liquid) and purified as described in EXAMPLE 2. The title compound was obtained as a white powder, 49.0 mg.
FAB $(M+H)^+$ at M/Z: 1669
AMINO ACID ANALYSIS: Arg(3.2), Asx(1.0), Cys(1.1), Gly(2.0), Ile(2.0), Phe(1.9), Ala(0.9).
The 300 MHz NMR is consistent with the proposed structure.

EXAMPLE 83

Arginyl-Cysteinyl-Glycyl-2-Aminoindane-2-Carbonyl-Glycyl-Glycyl-Arginyl-Isoleucyl-Aspartyl-Arginyl-Isoleucyl-Cysteinyl-Phenylalanyl-Arginine Cyclic Disulfide

0.56 g Protected peptide resin, prepared from N-a-t-Boc-Arg-(Ng-Tos) Merrifield resin and the amino acids added sequentially in the following order: Boc-Phe, Boc-Cys(4-MeBzl), Boc-Ile, Boc-Arg(Ng-Tos), Boc-Asp(β-cyclohexyl), Boc-Ile, Boc-Arg(Ng-Tos), Boc-Gly, Boc-Gly, Boc-Aic, Boc-Gly, Boc-Cys(4-MeBzl), Boc-Arg(Ng-Tos), was treated with HF (liquid) and purified as described in EXAMPLE 2. The title compound was obtained as a white powder, 31 mg.
FAB $(M+H)^+$ at M/Z: 1665
AMINO ACID ANALYSIS: Arg(4.0), Asx(1.0), Cys(2.0), Gly(2.9), Ile(2.1), Phe(1.2), Ser(1.4).
The 500 MHz NMR is consistent with the proposed structure.

EXAMPLE 84

Seryl-Seryl-Cysteinyl-D-Alanyl-Phenylalanyl-Glycyl-Glycyl-Arginyl-Isoleucyl-Aspartyl-Arginyl-Isoleucyl-4-Mercapto-3-aminobutanoyl-Phenylalanyl-Arginyl Cyclic Disulfide

0.70 g Protected peptide resin, prepared from N-a-t-Boc-Arg-(Ng-Tos) Merrifield resin and the amino acids added sequentially in the following order: Boc-Phe, Boc-hCys(4-MeBzl), Boc-Ile, Boc-Arg(Ng-Tos), Boc-Asp(β-cyclohexyl), Boc-Ile, Boc-Arg(Ng-Tos), Boc-Gly, Boc-Gly, Boc-Phe, Boc-D-Ala, Boc-Cys(4-MeBzl), Boc-Ser(Bzl), Boc-Ser(Bzl), was treated with HF (liquid) and purified as described in EXAMPLE 2. The title compound was obtained as a white powder, 53.4 mg.
FAB $(M+H)^+$ at M/Z: 1700
AMINO ACID ANALYSIS: Arg(3.2), Asx(1.1), Cys(0.4), Gly(2.2), Ile(1.7), Phe(2.0), Ala(1.0), Ser(1.4).
The 500 MHz NMR is consistent with the proposed structure.

EXAMPLE 85

Seryl-Seryl-Cysteinyl-D-Alanyl-Phenylalanyl-Glycyl-Glycyl-Arginyl-Isoleucyl-Aspartyl-Arginyl-Isoleucyl-Cis-4-Thiomethyl-Prolyl-Phenylalanyl-Arginyl Cyclic Disulfide

0.45 g Protected peptide resin, prepared from N-a-t-Boc-Arg(Ng-Tos) Merrifield resin and the amino acids added sequentially in the following order: Boc-Phe, Boc-Pro-cis-4-thiomethyl-S-(4-MeBzl), Boc-Ile, Boc-Arg(Ng-Tos), Boc-Asp(β-cyclohexyl), Boc-Ile, Boc-Arg(Ng-Tos), Boc-Gly, Boc-Gly, Boc-Phe, Boc-D-Ala, Boc-Cys(4-MeBzl), Boc-Ser(Bzl), Boc-Ser(Bzl), then was treated with HF (liquid) and purified as described in EXAMPLE 2. The title compound was obtained as a white powder, 60 mg.
FAB $(M+H)^+$ at M/Z: 1711
AMINO ACID ANALYSIS: Arg(3.0), Asx(1.0), Cys(0.5), Gly(1.9), Ile(2.1), Phe(1.9), Ser(1.5), Ala(1.0).
The 500 MHz NMR is consistent with the proposed structure.

EXAMPLE 86

Seryl-Seryl-Cysteinyl-D-Alanyl-Phenylalanyl-Glycyl-Glycyl-Arginyl-Isoleucyl-Aspartyl-Arginyl-Isoleucyl-Trans-4-Thiomethyl-D-Prolyl-Phenylalanyl-Arginyl Cyclic Disulfide

0.45 g Protected peptide resin, prepared from N-a-t-Boc-Arg(Ng-Tos) Merrifield resin and the amino acids added sequentially in the following order: Boc-Phe, Boc-Pro-trans-4-thiomethyl-S-(4-MeBzl), Boc-Ile, Boc-Arg(Ng-Tos), Boc-Asp(β-cyclohexyl), Boc-Ile, Boc-Arg(Ng-Tos), Boc-Gly, Boc-Gly, Boc-Phe, Boc-D-Ala, Boc-Cys(4-MeBzl), Boc-Ser(Bzl), Boc-Ser(Bzl), then was treated with HF (liquid) and purified as described in EXAMPLE 2. The title compound was obtained as a white powder, 65 mg.
FAB (M+H)+ at M/Z: 1711
AMINO ACID ANALYSIS: Arg(3.0), Asx(1.1), Gly(1.9), Ile(2.1), Phe(2.0), Ser(1.7), Ala(0.9).
The 300 MHz NMR is consistent with the proposed structure.

EXAMPLE 87

Cysteinyl-D-Alanyl-Phenylalanyl-Glycyl-Glycyl-Arginyl-Isoleucyl-Aspartyl-Arginyl-Isoleucyl-Cysteinyl-D-Tetrahydroisoquinolinyl-Arginyl Carboxamide Cyclic Disulfide

0.45 g Protected peptide resin, prepared from p-Methylbenzhydryl amine resin and the amino acids added sequentially in the following order: Boc-Arg(Ng-Tos) Boc-D-Tic, Boc-Cys(4-MeBzl), Boc-Ile, Boc-Arg-(Ng-Tos), Boc-Asp(B-cyclohexyl), Boc-Ile, Boc-Arg(Ng-Tos), Boc-Gly, Boc-Gly, Boc-Phe, Boc-D-Ala, Boc-Cys(4-MeBzl), then was treated with HF (liquid) and purified as described in EXAMPLE 2. The title compound was obtained as a white powder, 16 mg.
FAB $(M+H)^+$ at M/Z: 1522
AMINO ACID ANALYSIS: Arg(2.9), Asx(1.0), Cys(1.1), Gly(2.1), Ile(2.0), Phe(1.2), Ser(0.4), Ala(0.9).
The 500 MHz NMR is consistent with the proposed structure.

EXAMPLE 88

Seryl-Seryl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-HomoArginyl Cyclic Disulfide

0.45 g Protected peptide resin, prepared from N-a-t-Boc-Lys(2-Cl-Z) Merrifield resin and the amino acids added sequentially in the following order: Boc-Phe, Boc-Cys(4-MeBzl), Boc-Ile, Boc-Arg(Ng-Tos), Boc-Asp(β-cyclohexyl), Boc-Ile, Boc-Arg(Ng-Tos), Boc-Gly, Boc-Gly, Boc-Phe, Boc-D-Ala, Boc-Cys(4-MeBzl), Boc-Ser(Bzl), Boc-Ser(Bzl), then guanidylation was performed in the manner described for example 92, then was treated with HF (liquid) and purified as described in EXAMPLE 2. The title compound was obtained as a white powder, 5 mg.
FAB $(M+H)^+$ at M/Z: 1686
AMINO ACID ANALYSIS: Arg(1.9), Asx(1.0), Cys (1.4), Gly(3.1), Ile(1.9), Phe(2.1), Ser(1.4).

## EXAMPLE 89

Seryl-Cysteinyl-(D)Alanyl-Phenylalanyl-Glycyl-Glycyl-Arginyl-Isoleucyl-Aspartyl-Arginyl-Isoleucyl-Cysteinyl-Phenylalanyl-Glutaminyl Cyclic Disulfide

0.45 g Protected peptide resin, prepared from N-a-t-Boc-Gln Merrifield resin and the amino acids added sequentially in the following order: Boc-Phe, Boc-Cys(4-MeBzl), Boc-Ile, Boc-Arg(Ng-Tos), Boc-Ap(β-cyclohexyl), Boc-Ile, Boc-Arg(Ng-Tos), Boc-Gly, Boc-Gly, Boc-Phe, Boc-D-Ala, Boc-Cys(4-MeBzl), Boc-Ser-(Bzl), then was treated with HF (liquid) and purified as described in EXAMPLE 2. The title compound was obtained as a white powder, 11 mg.
FAB $(M+H)^+$ at M/Z: 1570
AMINO ACID ANALYSIS: Arg(2.1), Asx(0.9), Cys (1.0), Gly(1.8), Ile(2.1), Phe(1.9), Ser(0.5), Ala(1.0), Glu-(1.1).
The 500 MHz NMR is consistent with the proposed structure.

## EXAMPLE 90

Acetyl-Arginyl-Cysteinyl-D-Alanyl-Phenylalanyl-Glycyl-Glycyl-Arginyl-Leucyl-Aspartyl-Arginyl-Isoleucyl-Cysteinyl-Phenylalanyl-Arginyl Cyclic Disulfide.

Example 2 the above peptide was acetylated as described for example 30; The starting peptide is prepared as described in example 4.
FAB (M+H)+ at M/Z: 1710
AMINO ACID ANALYSIS: same as that found in example 4.
The 300 MHz NMR is consistent with the proposed structure.

## EXAMPLE 91

Carbamoyl-Seryl-Seryl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.45 g Protected peptide resin, prepared from N-a-t-Boc-Arg-(Ng-Tos) Merrifield resin and the amino acids added sequentially in the following order: Boc-Phe, Boc-Cys(4-MeBzl), Boc-Ile, Boc-Arg(Ng-Tos), Boc-Asp(β-cyclohexyl), Boc-Ile, Boc-Arg(Ng-Tos), Boc-Gly, Boc-Gly, Boc-Phe, Boc-D-Ala, Boc-Cys(4-MeBzl), Boc-Ser(Bzl), Boc-Ser(Bzl), then carbamoylation was performed with potassium cyanate in water and resin was treated with HF (liquid) and purified as described in example 2. The title compound was obtained as a white powder, 3 mg.
FAB $(M+H)^+$ at M/Z: 1686
AMINO ACID ANALYSIS: Arg(1.9), Asx(1.1), Cys (1.4), Gly(3.0), Ile(1.9) Phe(1.9), Ser(0.9), Lys(1.0).

## EXAMPLE 92

Guanido-Seryl-Seryl-Cysteinyl-D-Valyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

To a solution of the above peptide (10.7 mg; example 26) in DMF (1.4 mL) was added 1-guanyl-3.5-dimethyl pyrazole nitrate (11.7 mg) and this solution was stirred at room temperature for 35 days, then diluted with aqueous TFA (0.1% v/v; 4 mL) and purified as described in EXAMPLE 2. The title compound was obtained as a white powder, 2.8 mg.
FAB (M+H)+ at M/Z: 1757
AMINO ACID ANALYSIS: Arg(3.2), Asx(1.0), Cys(1.6), Gly(2.2), Ile(2.0), Phe(2.1), Ser(1.0), Val(0.9).
The 300 MHz NMR is consistent with the proposed structure.

## EXAMPLE 93

Guanido-8-Aminooctanoyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

To a solution of the above peptide (13.7 mg; example 45) in DMF (1.3 mL) was added 1-guanyl-3,5-dimethyl pyrazole nitrate (27.3 mg) and this solution was stirred at room temperature for 12 days, then diluted with aqueous TFA (0.1% v/v; 4 mL) and purified as described in EXAMPLE 2. The title compound was obtained as a white powder, 6.0 mg.
FAB $(M+H)^+$ at M/Z: 1694
AMINO ACID ANALYSIS: Arg(3.1), Asx(1.0), Cys(1.7), Gly(2.0), Ile(2.0), Phe(2.0), Ala(1.0)
The 300 MHz NMR is consistent with the proposed structure.

## EXAMPLE 94

Guanido-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

Example 55 the above peptide was guanidylated as described for example 92; The starting peptide is prepared as described in example 55.
FAB (M+H)+ at M/Z: 1555
AMINO ACID ANALYSIS: same as that found in example 55.
The 300 MHz NMR is consistent with the proposed structure.

## EXAMPLE 95

Guanido-Aminohexanoyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

Example 46 the above peptide was guanidylated as described for example 92; The starting peptide is prepared as described in example 46.
FAB $(M+H)^+$ at M/Z: 1711
AMINO ACID ANALYSIS: same as that found in example 46.
The 300 MHz NMR is consistent with the proposed structure.

EXAMPLE 96

trans-4-Aminomethylcyclohexoyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.82 g Protected peptide resin, prepared from N-a-t-Boc-Arg-(Ng-Tos) Merrifield resin and the amino acids added sequentially in the following order: Boc-Phe, Boc-Cys(4-MeBzl), Boc-Ile, Boc-Arg(Ng-Tos), Boc-Asp(β-cyclohexyl), Boc-Ile, Boc-Arg(Ng-Tos), Boc-Gly, Boc-Gly, Boc-Phe Boc-(D)-Ala, Boc-Cys(4-MeBzl), Boc-Amcca, was treated with HF (liquid) and purified as described in example 2. The title compound was obtained as a white powder, 28.1 mg.
FAB $(M+H)^{+}$ at M/Z: 1652.
AMINO ACID ANALYSIS: Arg(3.0), Asx(1.0), Cys(1.7), Gly(2.0), Ile(2.0), Phe(1.9), Ala(1.0).
The 300 MHz NMR is consistent with the proposed structure.

EXAMPLE 97

Guanido-trans-4-Aminomethylcyclohexoyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

To a solution of the above peptide A-68516 (9.0 mg; example) in DMF (1.0 mL) was added 1-guanyl-3,5-dimethyl pyrazole nitrate (18.0 mg) and this solution was stirred at room temperature for 10 days, when diluted with aqueous TFA (0.1% v/v; 4 mL) and purified as described in example 2. The title compound was obtained as a white powder, 3.5 mg.
FAB (M+H)+ at M/Z: 1692.
AMINO ACID ANALYSIS: Arg(3.0), Asx(1.0), Cys(0.5), Gly(2.1), Ile(2.0), Phe(1.9), Ala(1.1).

EXAMPLE 98

4-Aminobutanoyl-Cysteinyl-β-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.85 g Protected peptide resin, prepared from N-alpha-t-Boc-Arg($N^G$-Tos) Merrifield resin and the amino acids added sequentially in the following order: Boc-Phe, Boc-Cys(4-MeBzl), Boc-Ile, Boc-Arg($N^G$-Tos), Boc-Asp(β-cyclohexyl), Boc-Ile, Boc-Arg($N^G$-Tos), Boc-Gly, Goc-Gly, Boc-Phe, Boc-B-Ala, Boc-Cys(4-MeBzl), Boc-Abuta, was treated with HF (liquid) and purified as described in example 2. The title compound was obtained as a white powder, 17.5 mg.
FAB $(M+H)^{+}$ at M/Z: 1597.
AMINO ACID ANALYSIS: Arg(3.1), Asx(1.0), Cys(1.4), Gly(2.1), Ile(1.8), Phe(2.0), .
The 300 MHz NMR is consistent with the proposed structure.

EXAMPLE 99

Para-Aminophenylacetyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.84 g Protected peptide resin, prepared from N-a-t-Boc-Arg-(Ng-Tos) Merrifield resin and the amino acids added sequentially in the following order: Boc-Phe, Boc-Cys(4-MeBzl), Boc-Ile, Boc-Arg(Ng-Tos), Boc-Asp(β-cyclohexyl), Boc-Ile, Boc-Arg(Ng-Tos), Boc-Gly, Boc-Gly, Boc-Phe, Boc-D-Ala, Boc-Cys(4-MeBzl), Boc-Papaa, was treated with HF (liquid) and purified as described in EXAMPLE 2. The title compound was obtained as a white powder, 13.9 mg.
FAB (M+H)+ at M/Z: 1644.
AMINO ACID ANALYSIS: Arg(3.2), Asx(1.0), Cys(1.3), Gly(2.0), Ile(2.0), Phe(1.9), Ala(1.0).
The 300 MHz NMR is consistent with the proposed structure.

## EXAMPLE 100

Meta-Aminomethylbenzoyl-Cysteinyl-D-Alanyl-Phenylalanyl-Glycyl-Glycyl-Arginyl-Isoleucyl-Aspartyl-Arginyl-Isoleucyl-Cysteinyl-Phenylalanyl-Arginyl Cyclic Disulfide

0.71 g Protected peptide resin, prepared from N-a-t-Boc-Arg-(Ng-Tos) Merrifield resin and the amino acids added sequentially in the following order: Boc-Phe, Boc-Cys(4-MeBzl), Boc-Ile, Boc-Arg(Ng-Tos), Boc-Asp(β-cyclohexyl), Boc-Ile, Boc-Arg(Ng-Tos), Boc-Gly, Boc-Gly, Boc-Phe, Boc-D-Ala, Boc-Cys(4-MeBzl), Boc-Mamba, was treated with HF (liquid) and purified as described in example 2. The title compound was obtained as a white powder, 22.9 mg.
FAB $(M+H)^+$ at M/Z: 1645.
AMINO ACID ANALYSIS: Arg(3.0), Asx(1.0), Cys(1.1), Gly(1.9), Ile(2.0), Phe(2.0), Ala(1.0).
The 500 MHz NMR is consistent with the proposed structure.

## Vasorelaxant Activity

Female New Zealand white rabbits (2-3 kg) were anesthetized with phenobarbital sodium (30 mg/kg), and the descending thoracic aorta removed. The aorta was immediately placed in physiological saline (PSS) of the following millimolar composition: NaCl 119, KCl 4.7, $CaCl_2$ 2.5, $MgSO_4$ 1.5, $NaHCO_3$ 25, $KH_2PO_4$ 1.2, dextrose 11, $Na_2EDTA$ 0.03. After removal of surrounding tissue, rings 3 mm in length were mounted vertically in 2 ml tissue baths, and attached to Harvard 9529 transducers with 1.5 g resting tension in PSS at 37° C. Rings were equilibrated 15 minutes, exposed to (-)norepinephrine, 1 x $10^{-5}$ M, for 2 minutes, then washed with PSS. After a one hour interval of equilibration with PSS washes every 15 minutes, the tissues were contracted with histamine, 6 x $10^{-6}$ M. Cumulative additions of six half-log concentration increments of test compounds to each bath were begun 30 minutes after histamine addition. Dilutions of test compounds were made in 50mM sodium phosphate pH 7.4 containing 0.1% bovine serum albumin (vehicle). In each study, the vasorelaxant responses to vehicle and to the alpha-hANP were observed in control tissues. The negative log of the concentration which produced half-maximal relaxation (pD2) and the maximum vasorelaxant effect (Emax) relative to alpha-hANP are reported. The results of the testing are shown in Table I.

TABLE I

Vasorelaxant Activity

| Example No. | Agonist Testing (mean pD2) |
|---|---|
| 2 | 8.51 |
| 3 | 5.00 |
| 4 | 6.48 |
| 5 | 6.04 |
| 6 | 6.70 |
| 7 | 5.00 |
| 8 | 6.52 |
| 9 | 6.77 |
| 10 | 6.77 |
| 11 | 6.04 |
| 12 | 7.06 |
| 13 | 6.80 |
| 14 | 6.74 |
| 15 | 6.50 |
| 16 | 7.24 |
| 17 | 6.00 |
| 18 | 6.61 |
| 19 | 5.00 |
| 20 | 5.84 |
| 21 | 6.11 |
| 22 | 6.22 |
| 23 | 5.00 |
| 24 | 6.86 |
| 25 | 6.36 |
| 26 | 7.62 |
| 27 | 6.37 |
| 28 | 6.46 |
| 29 | 6.92 |
| 30 | 6.47 |
| 31 | 7.33 |
| 32 | 5.98 |
| 33 | 5.74 |
| 34 | 5.00 |
| 35 | 5.00 |
| 36 | 6.21 |
| 37 | 5.99 |
| 38 | 6.00 |
| 39 | 6.20 |
| 40 | 6.00 |
| 41 | 5.98 |
| 42 | 6.70 |
| 43 | 5.00 |
| 44 | 5.00 |
| 45 | 6.99 |
| 46 | 6.42 |

TABLE I continued

| Example No. | Agonist Testing (mean pD2) |
|---|---|
| 47 | 6.11 |
| 48 | 6.60 |
| 49 | 6.00 |
| 50 | 6.47 |
| 51 | 6.00 |
| 52 | 6.79 |
| 53 | 6.14 |
| 54 | 6.00 |
| 55 | 6.67 |
| 57 | 6.00 |
| 61 | 6.04 |
| 62 | 5.83 |
| 63 | 6.59 |
| 64 | 6.00 |
| 65 | 5.00 |
| 66 | 5.00 |
| 67 | 5.93 |
| 68 | 6.00 |
| 69 | 6.00 |
| 70 | 5.99 |
| 71 | 5.00 |
| 72A | 5.00 |
| 72B | 5.00 |
| 73 | 6.00 |
| 74 | 6.00 |
| 75 | 6.64 |
| 76 | 5.00 |
| 79 | 5.00 |

The foregoing is merely illustrative of the invention and is not intended to limit the invention to the disclosed compounds. Variations and changes which are obvious to one skilled in the art are intended to be within the scope and nature of the invention which are defined in the appended claims.

## Claims

1. A compound of the formula:

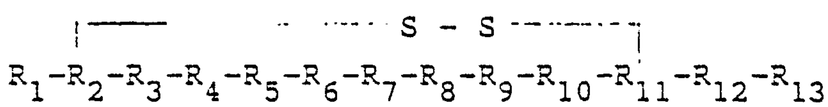

wherein $R_1$ is hydrogen, Ser, Cit, SerSer, Arg, Carbamoyl Ser, Guanido-Ser-Ser, Amcca, Guanido-Amcca, Lys, ArgArg, Ser-Arg, Ser-Lys, Acetyl-Ser-Ser, D-Ser-Ser, Ser-D-Ser, Guanido-Aocta, Aunda, Aocta, Ahexa, HSer, Apenta, Papaa, Abuta, Mamba, Orn, D-Orn or Acetyl-apenta, Acetyl Ahexa, Guanido;
$R_2$ is Cys, D-Cys or Pen;
$R_3$ is L-Phe, D-Ala, L-Ala, D-Val, D-Cha, Sar, D-Phg, Aib, β-Ala or Gly;
$R_4$ is Phe, Trp, D-Tic, L-Tic, N-MePhe, D-Ala, Tyr, 2-Thi, DimeDOPA or DOPA;
$R_5$ is Gly-Gly, Ala-Gly, D-Ala-Gly, Gly-Ala, Amcca, Mapaa or Papaa;
$R_6$ is Arg, D-Arg or Cit;
$R_7$ is Ile, Leu, D-Leu or Met;

$R_8$ is Asp, Asn, $\beta$-methylAsp, cycloAsp, Gly or Ser;
$R_9$ is Arg, D-Arg or Ala;
$R_{10}$ is Ile, Leu or Phe;
$R_{11}$ is Cys, D-Cys, HCys, Cys-4-thiomethylproline, or trans-4-thiomethylproline;
$R_{12}$ is absent, Phe, D-Ala-Phe, D-Phe, Cha, Pro, D-Pro, 2-Thi, Aic, D-Tic, trans-4-FPro, L-Tic, Leu, Ile or $\beta$-NAl;
$R_{13}$ is OH, Arg, Arg-$NH_2$, D-Arg, Gln, Lys, HomoArg, or Gly; or pharmaceutically acceptable salts, esters or amides thereof.

2. The compound of Claim 1 wherein $R_1$ is Ser-Ser, Arg, Ser-Arg, Lys, Aocta, or Aunda; $R_2$ is Cys or D-Cys; $R_3$ is D-Ala, Gly, D-Val or D-Cha; $R_4$ is Phe, Trp, Tic or N-MePhe; $R_5$ is Gly-Gly, D-Ala-Gly or Mapaa; $R_6$ is Arg; $R_7$ is Ile, Leu or Met; $R_8$ is Asp; $R_9$ is Arg; $R_{10}$ is Ile; $R_{11}$ is Cys; $R_{12}$ is Phe, Cha, Leu, D-Phe, Pro, Tic or D-Ala-Phe; and $R_{13}$ is Arg, Arg-$NH_2$ or D-Arg.

3. A composition for treating hypertension, producing natriuresis, producing diuresis, producing renovasodilation or providing renoprotection comprising a therapeutically effective amount of a compound of Claim 1 and a pharmaceutical carrier.

4. A method for treating hypertension, producing natriuresis, producing diuresis, producing renovasodilation or providing renoprotection comprising administering to a patient in need, a therapeutically effective amount of a compound of Claim 1.

5. A process for the preparation of a compound of the formula:

$$\overbrace{R_1-R_2-R_3-R_4-R_5-R_6-R_7-R_8-R_9-R_{10}-R_{11}}^{S\ -\ S}-R_{12}-R_{13}$$

wherein $R_1$ is hydrogen, Ser, Cit, SerSer, Arg, Carbamoyl Ser, Guanido-Ser-Ser, Amcca, Guanido-Amcca, Lys, Arg-Arg, Ser-Arg, Ser-Lys, Acetyl-Ser-Ser, D-Ser-Ser, Ser-D-Ser, Guanido-Aocta, Aunda, Aocta, Ahexa, HSer, Apenta, Papaa, Abuta, Mamba, Orn, D-Orn or Acetyl-apenta, Acetyl-Ahexa, Guanido;
$R_2$ is Cys, D-Cys or Pen;
$R_3$ is L-Phe, D-Ala, L-Ala, D-Val, D-Cha, Sar, D-Phg, Aib, $\beta$-Ala or Gly;
$R_4$ is Phe, Trp, D-Tic, L-Tic, N-MePhe, D-Ala, Tyr, 2-Thi, DimeDOPA or DOPA;
$R_5$ is Gly-Gly, Ala-Gly, D-Ala-Gly, Gly-Ala, Amcca, Mapaa or Papaa;
$R_6$ is Arg, D-Arg or Cit;
$R_7$ is Ile, Leu, D-Leu or Met;
$R_8$ is Asp, Asn, $\beta$-methylAsp, cycloAsp, Gly or Ser;
$R_9$ is Arg, D-Arg or Ala;
$R_{10}$ is Ile, Leu or Phe;
$R_{11}$ is Cys, D-Cys, HCys, Cys-4-thiomethylproline, or trans-4-thiomethylproline;
$R_{12}$ is absent, Phe, D-Ala-Phe, D-Phe, Cha, Pro, D-Pro, 2-Thi, Aic, D-Tic, trans-4-FPro, L-Tic, Leu, Ile or $\beta$-NAl;
$R_{13}$ is OH, Arg, Arg-$NH_2$, D-Arg, Gln, Lys, HomoArg, or Gly, comprising sequentially coupling suitable protected amino acid analogs, followed by removal of the protecting groups.

6. The process of Claim 5 wherein sequences of two or more suitably protected amino acids or amino acid analogs are coupled and the resulting peptides are coupled, followed by removal of the protecting groups, to provide the desired product.